# EUROPEAN PATENT APPLICATION

(11) **EP 4 837 901 A1**
(43) Date of publication of application: **11.11.2026**
(21) Application number: 24916510.1
(22) Date of filing: 18.10.2024
(51) Int. Cl.: C07D 277/46, A61K 31/454, A61P 35/00

(54) **PHENYLTHIAZOLAMINE-BASED PI4KIIIß INHIBITOR, PREPARATION METHOD THEREFOR, PHARMACEUTICAL COMPOSITION THEREOF AND USE THEREOF**

(30) Priority: 09.01.2024 CN 202410028674
(71) Applicant: Nanjing Hongshun Pharmaceutical Technology Co., Ltd, Nanjing, Jiangsu 210000 (CN); China Pharmaceutical University, Nanjing, Jiangsu 210000 (CN)
(72) Inventor: ZHOU, Qingfa, Nanjing, Jiangsu 210000 (CN); YAO, Shengfa, Nanjing, Jiangsu 210000 (CN); WANG, Enyuan, Nanjing, Jiangsu 210000 (CN); WANG, Bichuan, Nanjing, Jiangsu 210000 (CN)
(74) Representative: Bayramoglu et al.
(86) International application number: PCT/CN2024/125722
(87) International publication number: WO 2025/148459

(57) **Abstract**

The present invention discloses a phenylthiazolamine-based PI4KIIIβ inhibitor, which is a substituted phenylthiazolamine-based compound of Formula V, or a stereoisomer, hydrate or pharmaceutically acceptable salt thereof. The inhibitor has the characteristic of effectively inhibiting a PI3K/Akt/mTOR signaling pathway of PI4KIIIβ, and has efficient and excellent PI4KIIIβ enzyme inhibitory activity, low costs, high efficacy, low toxicity and high yield of intermediates during synthesis, thereby reducing resources waste and further facilitating cost reduction; and the inhibitor has a small dosage requirement and significant activity when applied to anti-tumor drugs.

## Description

### TECHNICAL FIELD

The present invention relates to a phenylthiazolamine-based PI4KIIIβ inhibitor, and further relates to a method for preparing the inhibitor, a pharmaceutical composition of the inhibitor and use of the inhibitor, belonging to the technical field of medicines.

### BACKGROUND TECHNOLOGY

Traditional therapies for malignant tumors mainly include surgery, chemotherapy and radiotherapy. Although the surgery is an effective method, it is not ideal for prolonging the life of a patient if other adjuvant therapies are not further carried out. The chemotherapy may lead to drug resistance and cause side effects, typically including bone marrow suppression, impaired intestinal function, etc. The radiotherapy is a localized therapy that can inhibit tumor growth, but may also affect and cause damage to surrounding tissues. A molecular targeted therapy has emerged as a new approach for treating cancer due to its high specificity and low toxicity.

Lipid phosphatidylinositol is an important regulator in numerous cellular processes, including signaling, membrane trafficking and cytokinesis. Phosphorylated phosphatidylinositol is produced through the phosphorylation of the inositol ring of phosphatidylinositol. Phosphatidylinositol can be phosphorylated and dephosphorylated by a set of different enzymes, resulting in a total of seven different mono-phosphorylated and poly-phosphorylated forms of phosphatidylinositols. Phosphatidylinositol 4-phosphate (PI4P), a lipid species, is produced under the action of phosphatidylinositol 4-kinases (PI4Ks). PI4P constitutes the dominant precursor for the biosynthesis of two poly-phosphorylated signaling lipids: phosphatidylinositol 4,5-bisphosphate (PIP2) and phosphatidylinositol 3,4,5-trisphosphate (PIP3). In mammals, there are four distinct PI4K enzymes: two type II enzymes (PI4KIIα and PI4KIIβ) and two type III enzymes (PI4KIIα and PI4KIIβ).

PI4KIIIβ is a peripheral membrane protein mainly located in the Golgi apparatus and the trans-Golgi network (TGN). It is crucial for mediating lipid transport cytokinesis, maintaining lysosomal identity and regulating membrane trafficking, together with Rab GTPases. The dysregulation of PI4KIIIβ is also associated with cancer. PI4KIIIβ is frequently amplified and oncogenically activated in tumors, and its expression can inhibit apoptosis of cancer cells. The loss of PI4KIIIβ isoforms induces apoptosis of cancer cells. Although PI4KIIβ has been confirmed as a novel cancer driver, there have been few reports on studies of specific PI4KIIIβ inhibitors for cancer treatment. So far, several PI4KIIIβ small-molecule inhibitors have been disclosed as effective antiviral drug candidates in preclinical studies. Many studies have confirmed the therapeutic potential of PI4KIIIβ inhibitors, but much less attention has been paid to exploring the use of PI4KIIIβ inhibitors as a chemotherapy for cancer treatment. Therefore, it is meaningful and valuable to develop a new class of PI4KIIIβ inhibitors and explore their applications in cancer treatment.

### CONTENT OF THE INVENTION

Object of the invention: An object of the present invention is to provide a high-activity phenylthiazolamine-based PI4KIIIβ inhibitor, and further to provide a method for preparing the inhibitor, a pharmaceutical composition of the inhibitor and use of the inhibitor.

The phenylthiazolamine-based PI4KIIIβ inhibitor of the present invention is a substituted phenylthiazolamine-based compound of Formula V, or a stereoisomer, hydrate or pharmaceutically acceptable salt thereof: 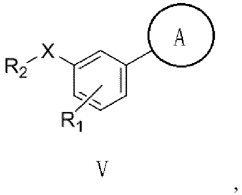 wherein A is 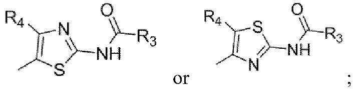 R₁ is a substituent on a benzene ring selected from the group consisting of hydrogen, fluorine, chlorine, bromine, iodine, hydroxy, amino, cyano, C1-C6 alkyl, C1-C6 haloalkyl, C1-C6 hydroxyalkyl, C1-C6 alkoxy, C1-C6 haloalkoxy, C1-C6 hydroxyalkoxy or C1-C6 alkoxy C1-C6 alkyl; R₂ is 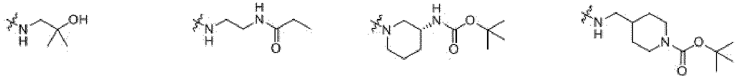 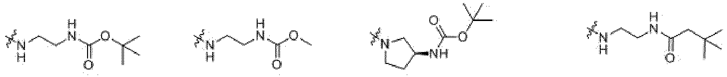 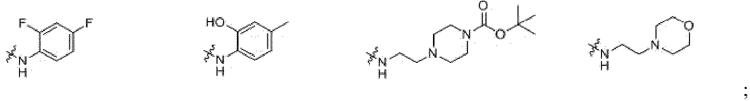 R₃ or R₄ is C1-C6 alkyl, C1-C6 alkyl substituted with one or more substituents, C1-C6 alkoxy, C1-C6 alkoxy substituted with one or more substituents, C1-C6 alkylacyl, C1-C6 alkylsulfonyl, C3-C6 heterocyclyl, or C3-C6 heterocyclyl substituted with one or more substituents; and
X is sulfonyl or carbonyl.

Preferably, the inhibitor is a compound of Formula I or II: 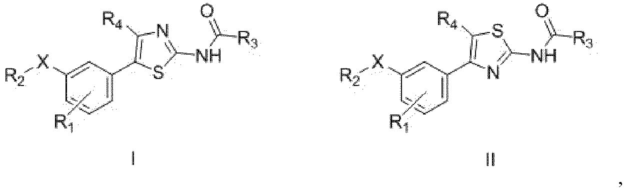 wherein R₁, R₂, R₄ and X are defined as 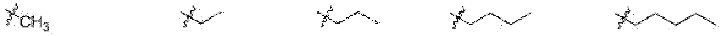 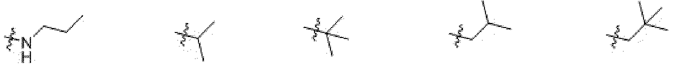 above, and R₃ is 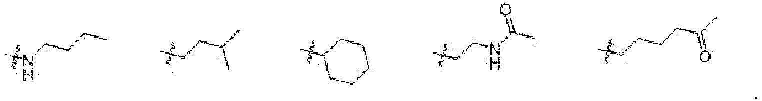 Preferably, in the inhibitor of Formula V, hydrogen attached to carbon is replaced by deuterium, which is an isotope of the hydrogen.

Further preferably, the alkyl group is replaced by a deuterated alkyl group, the alkoxy group is replaced by a deuterated alkoxy group, the benzene ring is replaced by a deuterated benzene ring, and the aromatic ring is replaced by a deuterated aromatic ring.

Preferably, the pharmaceutically acceptable salt refers to a salt form into which a basic group in the parent compound is converted, wherein the pharmaceutically acceptable salt is an inorganic or organic acid salt of a basic group, further preferably an amido or amino group; and the basic group in the parent compound reacts with 1 to 4 equivalents of an acid in a solvent system.

Preferably, the basic group of the compound of the present invention may form a salt with an acid, and the acid salt is specifically selected from: a salt formed with an inorganic acid, in particular a hydrohalic acid (such as hydrochloric acid, hydrobromic acid, or hydroiodic acid), nitric acid, sulfuric acid, phosphoric acid, carbonic acid, etc.; a salt formed with a lower alkylsulfonic acid, such as methanesulfonic acid or trifluoromethanesulfonic acid; a salt formed with an arylsulfonic acid, such as benzenesulfonic acid or p-toluenesulfonic acid; a salt formed with an organic acid, such as acetic acid, fumaric acid, tataric acid, oxalic acid, citric acid, maleic acid, malic acid or succinic acid; or a salt formed with an amino acid, such as aspartic acid or glutamic acid.

Preferably, the compound of the present invention and the pharmaceutically acceptable salt thereof further include a solvate or hydrate form.

Preferably, the structural formula of the compound in the phenylthiazolamine-based PI4KIIIβ inhibitor of the present invention includes isomeric forms, such as enantiomers, diastereomers, geometric isomers or conformational isomers, in particular R or S configurations containing asymmetric centers, *(Z)* or *(E)* isomers of double bonds, and *(Z)* or *(E)* conformational isomers.

Preferably, the inhibitor is one of:
(1) tert-butyl (2-(5-(2-acetamido-4-methylthiazol-5-yl)-2-chlorophenylsulfonamido)ethyl)carbamate;
(2) N-(5-(4-chloro-3-(N-(2-hydroxy-2-methylpropyl)aminosulfonyl)phenyl)-4-methylthiazol-2-yl)acetamide;
(3) N-(2-(2-acetamido-4-methylthiazol-5-yl)-2-chlorophenylsulfonamido)ethylpropionamide;
(4) methyl (2-(2-acetamido-4-methylthiazol-5-yl)-2-chlorophenylsulfonamido)ethyl carbamate;
(5) tert-butyl (2-((5-(2-acetamido-4-methylthiazol-5-yl)-2-methoxyphenyl)sulfonamido)ethyl)carbamate;
(6) N-(5-(4-chloro-3-(N-(3-hydroxy-4-methylphenyl)aminosulfonyl)phenyl)-4-methylthiazol-2-yl)acetamide;
(7) N-(5-(3-(N-(2.4-difluorophenyl)aminosulfonyl)-4-methoxyphenyl)-4-methylthiazol-2-yl)acetamide;
(8) tert-butyl (5-(2-propionamido-4-methylthiazol-5-yl)-2-methoxyphenylsulfonamido)ethyl carbamate;
(9) tert-butyl (5-(2-butanamido-4-methylthiazol-5-yl)-2-methoxyphenylsulfonamido)ethyl carbamate;
(10) tert-butyl (5-(2-pentanamido-4-methylthiazol-5-yl)-2-methoxyphenylsulfonamido)ethyl carbamate;
(11) tert-butyl (5-(2-hexanamido-4-methylthiazol-5-yl)-2-methoxyphenylsulfonamido)ethyl carbamate;
(12) tert-butyl (5-(2-heptanamido-4-methylthiazol-5-yl)-2-methoxyphenylsulfonamido)ethyl carbamate;
(13) tert-butyl (2-(2-methoxy-5-(4-methyl-2-(3-propylureido)thiazol-5-yl)phenyl)sulfonamido)ethyl carbamate;
(14) tert-butyl (2-(5-(2-(3-butylureido)-4-methylthiazol-5-yl)-2-methoxyphenylsulfonamido)ethyl)carbamate;
(15) tert-butyl (2-(2-methoxy-5-(4-methyl-2-(3-methylbutanamido)thiazol-5-yl)phenyl)sulfonamido)ethyl carbamate;
(16) tert-butyl (5-(2-isobutanamido-4-methylthiazol-5-yl)-2-methoxyphenylsulfonamido)ethyl carbamate;
(17) tert-butyl (2-(2-methoxy-5-(4-methyl-2-(2,2-dimethylpropanamido)thiazol-5-yl)phenyl)sulfonamido)ethyl carbamate;
(18) tert-butyl (5-(2-(3,3-dimethylbutanamido)-4-methylthiazol-5-yl)-2-methoxyphenylsulfonamido)ethyl carbamate;
(19) tert-butyl (2-(2-methoxy-5-(4-methyl-2-(4-methylpentanamido)thiazol-5-yl)phenyl)sulfonamido)ethyl carbamate;
(20) tert-butyl (2-(2-methoxy-5-(4-methyl-2-(5-methylhexanamido)thiazol-5-yl)phenyl)sulfonamido)ethyl carbamate;
(21) tert-butyl (2-(5-(2-(cyclohexanecarboxamido)-4-methylthiazol-5-yl)-2-methoxyphenyl)sulfonamido)ethyl carbamate;
(22) tert-butyl (2-(2-chloro-5-(4-methyl-2-(3-methylbutanamido)thiazol-5-yl)phenyl)sulfonamido)ethyl carbamate;
(23) tert-butyl (2-chloro-5-(2-(3,3-dimethylbutanamido)-4-methylthiazol-5-yl)phenylsulfonamido)ethyl carbamate;
(24) tert-butyl (2-chloro-5-(4-methyl-2-pentanamido-5-yl)phenylsulfonamido)ethyl carbamate;
(25) tert-butyl (2-chloro-5-(2-hexanamido-4-methylthiazol-5-yl)phenylsulfonamido)ethyl carbamate;
(26) tert-butyl (2-(5-(2-(3-acetamidopropionamido)-4-methylthiazol-5-yl)-2-methoxyphenyl)sulfonamido)ethyl carbamate;
(27) tert-butyl (2-(2-methoxy-5-(4-methyl-2-(6-oxoheptanamido)thiazol-5-yl)phenyl)sulfonamido)ethyl carbamate;
(28) tert-butyl (2-(2-methoxy-5-(4-methyl-2-(6-oxohexanamido)thiazol-5-yl)phenyl)sulfonamido)ethyl carbamate;
(29) tert-butyl (R)-(1-((5-(2-hexanamido-4-methylthiazol-5-yl)-2-methoxyphenyl)sulfonyl)piperidin-3-yl)carbamate;
(30) tert-butyl (S)-(1-((5-(2-hexanamido-4-methylthiazol-5-yl)-2-methoxyphenyl)sulfonyl)pyrrolidin-3-yl)carbamate;
(31) tert-butyl 4-((2-hexanamido-4-methylthiazol-5-yl)-2-methoxyphenylsulfonamido)piperidin-1-carboxylate;
(32) N-(5-(3-(2-(3,3-dimethylbutanamido)ethylaminosulfonyl)-4-methoxyphenyl)-4-methylthiazol-2-yl)hexanamide;
(33) N-(5-(3-(2-hydroxy-2-methylpropyl)aminosulfonyl)-4-methoxyphenyl)-4-methylthiazol-2-yl hexanamide;
(34) N-(5-(3-(N-(2-morpholinoethyl)aminosulfonyl))-4-methoxyphenyl)-4-methylthiazol-2-yl hexanamide;
(35) N-(5-(3-(((4-fluorophenyl)-12-nitridoacyl)sulfonyl)-4-methoxyphenyl)-4-methylthiazol-2-yl)hexanamide;
(36) N-(5-(3-(((4-chlorophenyl)-12-nitren)sulfonyl)-4-methoxyphenyl)-4-methylthiazol-2-yl)hexanamide;
(37) N-(5-(3-(((4-fluorobenzyl)-12-nitren)sulfonyl)-4-methoxyphenyl)-4-methylthiazol-2-yl)hexanamide;
(38) N-(5-(3-(((3-fluorobenzyl)-12-nitren)sulfonyl)-4-methoxyphenyl)-4-methylthiazol-2-yl)hexanamide;
(39) N-(5-(3-(((2-fluorobenzyl)-12-nitren)sulfonyl)-4-methoxyphenyl)-4-methylthiazol-2-yl)hexanamide;
(40) N-(4-(3-(((2,4-difluorobenzyl)-12-nitren)sulfonyl)-4-methoxyphenyl)-5-methylthiazol-2-yl)hexanamide;
(41) N-(5-(3-(((3,4-difluorobenzyl)-12-nitren)sulfonyl)-4-methoxyphenyl)-4-methylthiazol-2-yl)hexanamide;
(42) N-(5-(3-(((3-chloro-4-fluorobenzyl)-12-nitren)sulfonyl)-4-methoxyphenyl)-4-methylthiazol-2-yl)hexanamide;
(43) N-(5-(3-(((4-fluoro-3-(trifluoromethyl)benzyl)-12-nitren)sulfonyl)-4-methoxyphenyl)-4-methylthiazol-2-yl)hexanamide;
(44) N-(5-(4-methoxy-3-(((3.4.5-trifluorobenzyl)-12-nitren)sulfonyl)phenyl)-4-methylthiazol-2-yl)hexanamide;
(45) N-(5-(3-(((4-fluorophenethyl)-12-nitren)sulfonyl)-4-methoxyphenyl)-4-methylthiazol-2-yl)hexanamide;
(46) tert-butyl (2-(2-hexanamido-4-methylthiazol-5-yl)-2-methoxybenzamido)ethyl carbamate;
(47) N-(4-(3-(((2,4-difluorobenzyl)-12-nitren)sulfonyl)-4-methoxyphenyl)-5-methylthiazol-2-yl)hexanamide.

A method for preparing the inhibitor described above includes: reacting a starting material 1a, 1-(4-chlorophenyl)-2-acetone, or a starting material 1b, 1-(4-methoxyphenyl)-2-acetone, with chlorosulfonic acid to effect a meta-substitution reaction on the benzene ring, thereby obtaining an intermediate 2a or 2b, respectively; subjecting the intermediate 2a or 2b to a Hinsberg reaction to introduce an R₂ group, thereby obtaining intermediates 3a to 3g, respectively; subjecting the intermediates 3a to 3g and phenyltrimethylammonium tribromide to α-bromination, to obtain intermediates 4a to 4g, respectively; and condensing the intermediates 4a to 4g with N-acetylthiourea, to afford target compounds 5a to 5g, respectively, with a synthetic route depicted below: 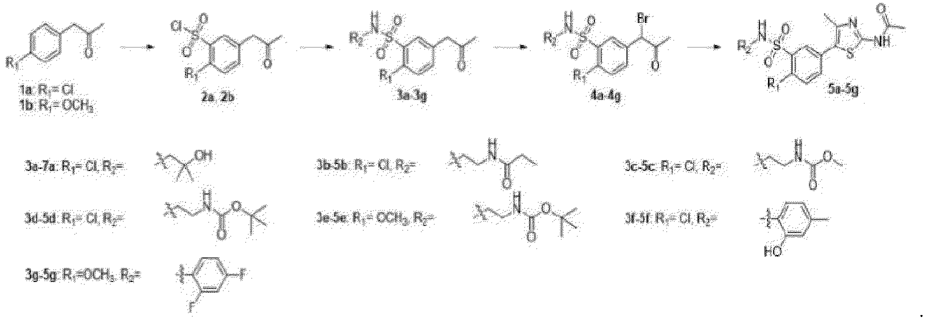 The method for preparing the inhibitor described above further includes: based on the method for preparing the target compounds 5a to 5g, reacting the intermediate 4d or 4e as a starting material with N-substituted thiourea as a further starting material, which is obtained by subjecting thiourea and a variety of acyl chlorides to a substitution reaction to introduce an R₃ group, to afford target compounds 7a to 7r, respectively, or condensing the intermediate 4e as a starting material with thiourea to obtain an intermediate 8 and subjecting the intermediate 8 and 3-acetamidopropanoyl chloride or 6-oxoheptanoyl chloride or 5-oxohexanoyl chloride to a substitution reaction, to afford target compounds 9a to 9c, respectively, with synthetic routes depicted below: 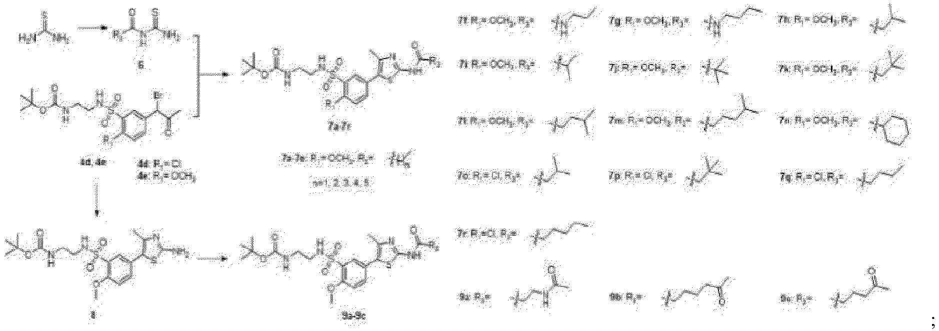 or based on the method for preparing the target compounds 5a to 5g, preparing intermediates 4h to 4x as starting materials and then condensing the intermediates 4h to 4x with N-hexanoylthiourea, to afford target compounds 10a to 10q, respectively, with a synthetic route depicted below: 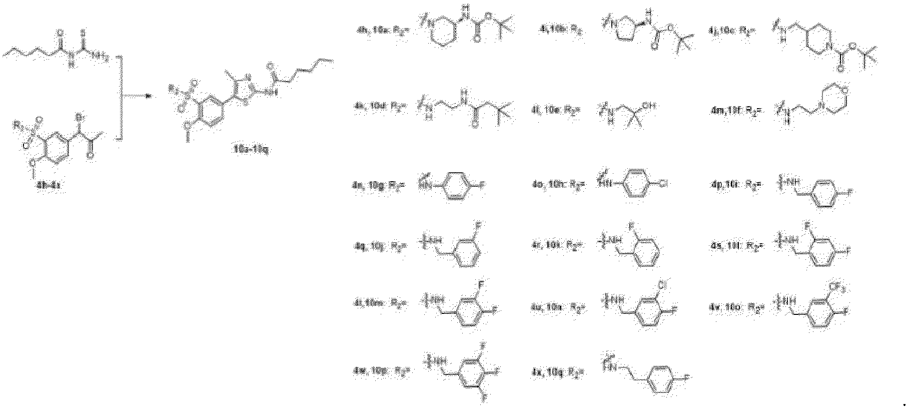 The method for preparing the inhibitor described above further includes: subjecting the intermediate 8 as a starting material to two sequential reactions to obtain an intermediate 9, subjecting the intermediate 9 and iron powder to a reduction reaction in refluxing acetic acid under heating conditions to obtain an intermediate 10, hydrolyzing the intermediate 10 under acidic conditions to obtain an intermediate 11, reacting the intermediate 11 with tert-butyl (2-aminoethyl)carbamate to obtain an intermediate 12, and subjecting the intermediate 12 to two sequential reactions to afford a target compound 46, with a synthetic route depicted below: 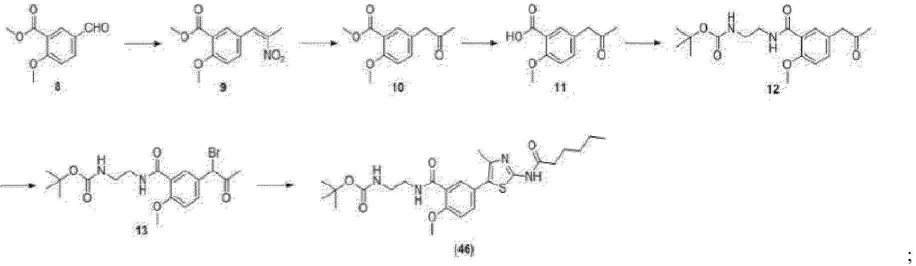 or subjecting 4'-methoxypropiophenone 14 as a starting material to four sequential reactions to afford a target compound 47, with a synthetic route depicted below:

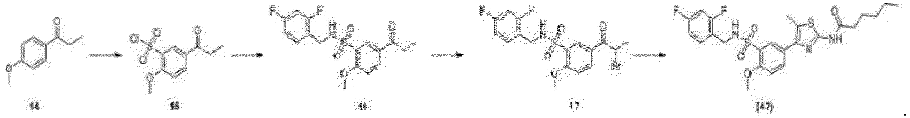

The present invention further discloses a pharmaceutical composition, including at least one pharmaceutically acceptable excipient, adjuvant or carrier, and the phenylthiazolamine-based PI4KIIIβ inhibitor described above.

The present invention further discloses use of the phenylthiazolamine-based PI4KIIIβ inhibitor or pharmaceutical composition described above in the preparation of a medicament for preventing, treating or adjunctively treating a proliferative disease, metabolic disease, neurological disorder or tuberous sclerosis complex caused by PI4KIIIβ hyperactivation.

Preferably, the proliferative disease includes colorectal cancer, gastric cancer, breast cancer, lung cancer, liver cancer, prostate cancer, pancreatic cancer, thyroid cancer, bladder cancer, kidney cancer, brain cancer, cervical cancer, cancer of the CNS, malignant glioma, myeloproliferative disease, blood cancer or lymphoma.

The present invention further discloses use of the phenylthiazolamine-based PI4KIIIβ inhibitor or pharmaceutical composition described above in the preparation of a medicament for inhibiting growth of cancer cells.

Principle of the invention: Due to low inhibitory activity and insufficient anti-tumor effect of PIK4KIIIβ, PIK-93 is selected as the lead compound for further structural modification. It is determined that phenylthiazolamine is used as a basic skeleton structure and substituent groups are screened, to bring about an efficient inhibitory effect.

Beneficial effects: Compared with the prior art, the present invention has the following significant advantages: (1) the characteristic of effectively inhibiting the PI3K/Akt/mTOR signaling pathway of PI4KIIIβ, and the efficient and excellent PI4KIIIβ inhibitory activity; (2) low cost, good efficacy, low toxicity and high yields of intermediate products during synthesis, resulting in reduced resource waste and further reduced cost; and (3) significant anti-tumor activity and low dosage requirement.

### DESCRIPTION OF THE DRAWINGS

FIG. 1 shows the results of in vivo anti-tumor efficacy and toxicity assessment for Examples 11 and 27, with A showing the curves of changes in tumor volume in mice, B showing the curves of body weight growth, and C showing the representative images of tumor tissues from mice;
FIG. 2 shows the results of in vivo anti-tumor efficacy and toxicity assessment for Example 40, with A showing the curves of changes in tumor volume in mice, B showing the curves of body weight growth, and C showing the representative images of tumor tissues from mice; and
FIG. 3 shows the images of stained histopathological sections of tumor-bearing mice.

### SPECIFIC IMPLEMENTATIONS

The technical solutions of the present invention are further described below in conjunction with examples and accompanying drawings.

Reagents were commercially available from commercial suppliers such as Anhui Zesheng Technology Co., Ltd., J&K Scientific, Aladdin Reagent Co., Ltd., Beijing Ouhe Technology Co., Ltd., etc., and were used without further purification, unless otherwise indicated. General reagents were commercially available from Xilong Chemical Co., Ltd., Nanjing Chemical Reagent Co., Ltd., Sinopharm Chemical Reagent Co., Ltd., Qingdao Haiyang Chemical Co., Ltd., etc. In the examples, all temperatures were set in degrees Celsius, unless otherwise indicated.

In the examples described below, chromatographic purification was performed using silica gel columns (200-300 mesh) commercially available from Qingdao Haiyang Chemical Co., Ltd. Nuclear magnetic resonance (NMR) spectra were recorded in a CDCl₃ or DMSO-*d₆* solvent (in ppm), using tetramethylsilane (TMS) at 0 ppm as internal reference. Peak multiplicities were denoted in abbreviation as follows: s (singlet), d (doublet), t (triplet), m (multiplet), br (broadened), dd (doublet of doublets), dt (doublet of triplets). Coupling constants were expressed in hertz (Hz).

In the examples described below, low-resolution mass spectrometry (MS) data were analyzed using a G1329B autosampler and a G4212B detector in an Agilent 6120 series LC-MS equipped with a G1311B quaternary pump and a G1316A column oven.

In the examples described below, for ease of description, some starting materials will be described by their abbreviations, which are illustrated below in contrast with their full names: DCM denotes CH₂Cl₂, i.e., dichloromethane; CDC1₃ denotes deuterated chloroform; PE denotes petroleum ether; both EtOAc and EA denote ethyl acetate; both MeOH and CH₃OH denote methanol; ClSO₃H denotes chlorosulfonic acid; both TEA and Et₃N denote triethylamine; DMSO-*d₆* denotes hexadeuterated dimethyl sulfoxide; THF denotes tetrahydrofuran; NaCl denotes sodium chloride; Na₂SO₄ denotes sodium sulfate; and CDI denotes N,N-carbonyldiimidazole.

### Example 1

Synthesis of tert-butyl (2-(5-(2-acetamido-4-methylthiazol-5-yl)-2-chlorophenylsulfonamido)ethyl)carbamate, with synthesis steps as below.

### Step 1: Synthesis of 2-chloro-5-(2-oxopropyl)benzenesulfonyl chloride

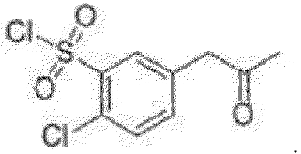

10 mL of ClSO₃H (132 mmol, 11.0 eq.) was volumetrically added to a 25 mL eggplant-shaped reaction flask and precooled in an ice bath for 30 min, and 2.02 g of (12.0 mmol) 4-chlorophenylacetone was slowly added dropwise to the ClSO₃H, allowing the reaction to proceed at room temperature for approximately 6 h, with completion confirmed by thin-layer chromatography (TLC) showing full consumption of a starting material 1a. A reaction mixture was slowly added dropwise to ice cubes to quench unreacted ClSO₃H; a resulting aqueous solution was extracted with ethyl acetate (3 × 100 mL) to collect organic phases; and the organic phases were washed with an aqueous saturated sodium chloride solution (50 mL), dried over anhydrous sodium sulfate, concentrated under reduced pressure to obtain a crude product; and the crude product was purified by column chromatography (PE:EA = 4:1 to 2:1). A white solid, yield: 60%. ¹H NMR (300 MHz, Chloroform-*d*) *δ* 7.95 (d,*J* = 2.1 Hz, 1H), 7.63 (d,*J* = 8.2 Hz, 1H), 7.50 (dd,*J* = 8.2, 2.1 Hz, 1H), 3.86 (s, 2H), 2.30 (s, 3H).Step 2: Synthesis of tert-butyl (2-(2-chloro-5-(2-oxopropyl)phenylsulfonamido)ethyl)carbamate

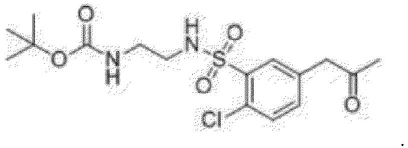

5 mmol 2-chloro-5-(2-oxopropyl)benzenesulfonyl chloride was metrologically weighed and dissolved in 20 mL of dichloromethane, 7.5 mmol tert-butyl (2-aminoethyl)carbamate (1.5 eq.) and 1.01 g of triethylamine (2.0 eq.) was added to a reaction mixture and stirred at room temperature for 12 h until full consumption of 2-chloro-5-(2-oxopropyl)benzenesulfonyl chloride. Upon completion of the reaction, a residue was concentrated and purified by column chromatography (PE:EA = 1:1). A white solid, yield: 78%. ¹H NMR (400 MHz, DMSO-*d₆*) *δ* 7.85 (t,*J* = 5.9 Hz, 1H), 7.78 (d,*J* = 2.1 Hz, 1H), 7.60 (d,*J* = 8.1 Hz, 1H), 7.43 (dd,*J* = 8.2, 2.2 Hz, 1H), 6.78 (t,*J* = 5.8 Hz, 1H), 3.95 (s, 2H), 3.02-2.93 (m, 2H), 2.84 (dt,*J* = 8.2, 6.0 Hz, 2H), 2.18 (s, 3H), 1.35 (s, 9H); ¹³C NMR (101 MHz, DMSO-*d₆*) δ 205.63, 155.97, 137.78, 135.79, 135.40, 132.09, 131.92, 129.09, 78.30, 48.54, 42.60, 30.21, 28.64.

### Step 3: Synthesis of tert-butyl (5-(1-bromo-2-(oxopropyl)-2-chlorophenylsulfonamido)ethyl)carbamate

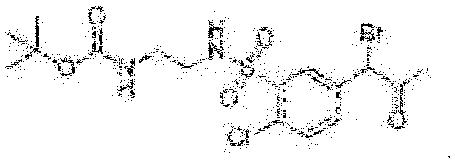

1 mmol tert-butyl (2-(2-chloro-5-(2-oxopropyl)phenylsulfonamido)ethyl)carbamate was metrologically weighed, dissolved in 5 mL of tetrahydrofuran solution and then contained in an eggplant-shaped flask; 10 mL of tetrahydrofuran solution containing 1.1 mmol phenyltrimethylammonium tribromide was slowly added dropwise to a reaction mixture from the above step in an ice bath; and upon completion of dropwise addition, the ice bath was removed, allowing the reaction to proceed at room temperature for 0.5 h until full consumption of the reactants; and a residue was purified (PE:EA = 1:1) to obtain a brominated product. Light yellow oil, yield: 60%. ¹H NMR (300 MHz, Chloroform-d) *δ* 8.11 (d,*J =* 2.3 Hz, 1H), 7.66 (dd,*J* = 8.3, 2.3 Hz, 1H), 7.57 (d,*J =* 8.3 Hz, 1H), 5.72 (s, 1H), 5.42 (s, 1H), 4.87 (s, 1H), 3.28 (q,*J* = 5.7 Hz, 2H), 3.08 (q,J= 5.8 Hz, 2H), 2.44 (s, 3H), 1.46 (s, 9H).
Step 4: Synthesis of tert-butyl (2-(5-(2-acetamido-4-methylthiazol-5-yl)-2-chlorophenylsulfonamido)ethyl)carbamate, with a structural formula as below:

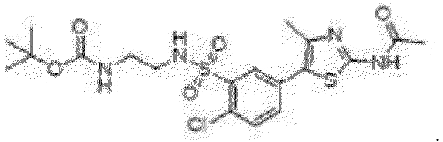

N-acetylthiourea was first prepared as follows: 10 mmol thiourea was dissolved in 30 mL of an anhydrous toluene solution and contained in a 100 mL eggplant-shaped flask; 15 mmol acetyl chloride was metrologically weighed and dissolved in 2 mL of anhydrous toluene and a resulting mixture was slowly added dropwise to a reactor; the reactants were heated to 110°C for refluxing, allowing the reaction to proceed for 5 h; and upon completion of the reaction, the reaction mixture was cooled to room temperature, concentrated, and then purified by column chromatography (PE:EA = 8:1) to obtain N-acetylthiourea as a white solid (yield: 71%). ¹H NMR (400 MHz, Chloroform-d) *δ* 9.88 (s, 1H), 8.99 (s, 1H), 7.14 (s, 1H), 2.19 (s, 3H). 1 mmol intermediate 4d was metrologically weighed and added to a 25 mL eggplant-shaped flask; 10 mL of an acetone solution was added; 1 mmol N-acetyl thiourea (1.0 eq.) was added to a resulting mixture; the mixture was heated to 60°C for refluxing, allowing the reaction to proceed for 1 h, with TLC monitoring the reaction until full consumption of the intermediate 4a (DCM:MeOH = 25:1); and a residue was concentrated in vacuum and purified by column chromatography. A white solid, yield: 60%. ¹H NMR (400M Hz, DMSO-d₆) *δ* 12.25 (s, 1H), 8.04 (t,*J =* 5.7 Hz, 1H), 7.93 (t,*J =* 1.3 Hz, 1H), 7.72 (d,*J =* 1.3 Hz, 2H), 6.77 (t,*J* = 5.6 Hz, 1H), 2.97 (q,*J* = 6.4 Hz, 2H), 2.93 - 2.80 (m, 2H), 2.37 (s, 3H), 2.15 (s, 3H), 1.33 (s.9H); ¹³C NMR (101 MHz, DMSO-d₆) *δ* 175.44, 156.03, 155.52, 143.80, 138.28, 133.23, 132.53, 131.84, 129.39, 128.96, 121.44, 77.86, 42.22, 42.21, 28.19, 19.09, 16.13. ESI-HRMS calcd for C₁₉H₂₅CIN₄O₅S₂ m/z [M+H]⁺ 489.1028, found [M+H]⁺ 489.1032.

### Example 2

N-(5-(4-chloro-3-(N-(2-hydroxy-2-methylpropyl)aminosulfonyl)phenyl)-4-methylthiazol-2-yl)acetamide, with a structural formula as below:

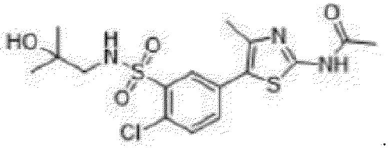

The fragment tert-butyl (2-aminoethyl)carbamate in Step 2 of Example 1 was replaced with 1-amino-2-methyl-2-propanol, while other steps and procedures were identical to those in Example 1. A white solid, yield: 25%. ¹H NMR (400 MHz, DMSO-*d₆*) *δ* 12.24 (s, 1H), 7.94 (s, 1H), 7.77 (t,*J* = 6.3 Hz, 1H), 7.72-7.68 (m, 2H), 4.45 (s, 1H), 2.83 (d,*J* = 6.2 Hz, 2H), 2.37 (s, 3H), 2.16 (s, 3H), 1.05 (s, 6H); ¹³C NMR (101 MHz, DMSO-*d₆*) *δ* 166.42, 145.50, 138.51, 132.58, 132.11, 132.03, 128.42, 127, 28, 114, 92, 68.87, 53.89, 27.03, 16.48. ESI-HRMS calcd for C₁₆H₂₀CIN₃O₄S₂ *m*/*z* [M+H]⁺ 418, 0657, found [M+H]⁺ 418.0663.

### Example 3

N-(2-(2-acetamido-4-methylthiazol-5-yl)-2-chlorophenylsulfonamido)ethyl propionamide, with a structural formula as below:

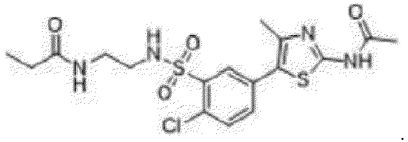

The fragment tert-butyl (2-aminoethyl)carbamate in Step 2 of Example 1 was replaced with N-(2-aminoethyl)propionamide, while other steps and procedures were identical to those in Example 1. A white solid, yield: 30%. ¹H NMR (400 MHz, DMSO-*d₆*) *δ* 7.93 (s, 1H), 7.76 (t,*J* = 5.7 Hz, 1H), 7.71-7.69 (m, 2H), 3.09 (q,*J* = 6.4 Hz, 2H), 2.92 (t,*J* = 6.7 Hz, 2H), 2.37 (s, 3H), 2.15 (s, 3H), 2.00 (q,*J* = 7.5 Hz, 2H), 0.93 (td,*J* = 7.6, 3H); ¹³C NMR (101 MHz, DMSO-*d₆*) *δ* 8173.13, 168.66, 155.88, 143.79, 138.26, 133.20, 132.48, 131.80, 129.34, 128.92, 121.34, 42.07, 38.62, 28.39, 22.48, 16.16, 9.74. ESI-HRMS calcd for C₁₇H₂₁CIN₄O₄S₂ *m*/*z* [M+H]⁺ 445.0766, found [M+H]⁺ 445.0762.

### Example 4

Methyl (2-(2-acetamido-4-methylthiazol-5-yl)-2-chlorophenylsulfonamido)ethyl carbamate, with a structural formula as below:

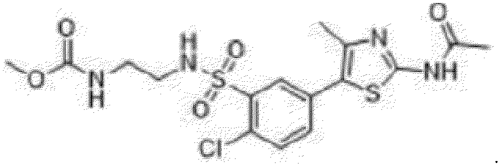

The fragment tert-butyl (2-aminoethyl)carbamate in Step 2 of Example 1 was replaced with methyl (2-aminoethyl)carbamate, while other steps and procedures were identical to those in Example 1. A white solid, yield: 29%. ¹H NMR (400 MHz, DMSO-*d₆*) *δ* 12.24 (s, 1H). 8.06 (t,*J* = 5.8 Hz, 1H). 7.93 (s, 1H), 7.71 (d,*J* = 1.3 Hz, 2H), 7.07 (t,*J* = 5.7 Hz, 1H), 3.46 (s, 3H), 3.03 (q,*J* = 6.4 Hz, 2H), 2.93 (q,*J* = 6.4 Hz, 2H), 2.37 (s, 3H), 2.15 (s, 3H); ¹³C NMR (101 MHz, DMSO-*d₆*) *δ* 168.66, 156.65, 155.90, 143.79, 138,28, 133.17, 132.47, 131.80, 129.33, 128.94, 121.38, 51.35, 42.12, 40.33, 22.48, 16.16. ESI-HRMS calcd for C₁₆H₁₉CIN₄O₅S₂ *m*/*z* [M+H]⁺ 447.0558, found [M+H]⁺ 447.0556.

### Example 5

Tert-butyl (2-((5-(2-acetamido-4-methylthiazol-5-yl)-2-methoxyphenyl)sulfonamido)ethyl)carbamate, with a structural formula as below:

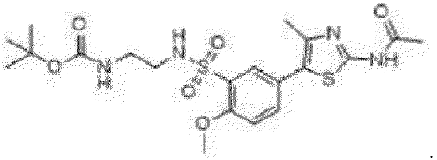

The starting material 1-(4-chlorophenyl)-2-acetone in Step 1 of Example 1 was replaced with 1-(4-methoxyphenyl)-2-acetone, while other steps and procedures were identical to those in Example 1. A white solid, yield: 28%. ¹H NMR (400 MHz, DMSO-*d₆*) *δ* 12.14 (s, 1H), 7.71 (d,*J* = 2.4 Hz, 1H), 7.68 (dd,*J* = 8.6, 2.4 Hz, 1H), 7.38 (t,*J* = 5.9 Hz, 1H), 7.31 (d,*J* = 8.6 Hz, 1H), 6.74 (t,*J* = 5.8 Hz, 1H), 3.94 (s, 3H), 2.95 (q,*J* = 6.5 Hz, 2H), 2.82 (q,*J* = 6.5 Hz, 2H), 2.32 (s, 3H), 2.14 (s, 3H), 1.34 (s, 9H); ¹³C NMR (101 MHz, DMSO-*d₆*) *δ* 168.41, 155.46, 155.30, 155.01, 142.06, 134.26, 128,89, 128.17, 124.02, 122.27, 113.62, 77.80, 56.37, 42.38, 42.36, 28.19, 22.46, 15.85; ESI-HRMS calcd for C₂₀H₂₈N₄O₆S₂ *m*/*z* [M+H]⁺ 485.1523, found [M+H]⁺ 485.1519.

### Example 6

N-(5-(4-chloro-3-(N-(3-hydroxy-4-methylphenyl)aminosulfonyl)phenyl)-4-methylthiazol-2-yl)acetamide, with a structural formula as below:

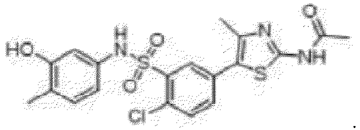

The fragment tert-butyl (2-aminoethyl)carbamate in Step 2 of Example 1 was replaced with 5-amino-2-methylphenol, while other steps and procedures were identical to those in Example 1. A white solid, yield: 25%. ¹H NMR (400 MHz, DMSO-*d₆*) *δ* 12.20 (s, 1H), 9.89 (s, 1H), 9.72 (s, 1H), 7.84 (dd,*J* = 10.3, 2.1 Hz, 1H), 7.73-7.61 (m, 2H), 7.25 (s, 1H), 6.72 (s, 1H), 5.75 (d,*J* = 1.6 Hz, 1H), 2.22 (d,*J* = 4.8 Hz, 3H), 2.18-2.09 (m, 6H); ¹³C NMR (101 MHz, DMSO-*d₆*) *δ* 168.58, 155.90, 150.55, 143.61, 138.06, 135.64, 133.50, 132.38, 131.30, 129.72, 129.51, 128.46, 122.67, 121.26, 117.71, 111.63, 22.44, 21.99, 15.78; ESI-HRMS calcd for C₁₉H₁₈CIN₃O₄S₂ *m*/*z* [M+H]⁺ 452.0500, found [M+H]⁺ 452.0511.

### Example 7

N-(5-(3-(N-(2,4-difluorophenyl)aminosulfonyl)-4-methoxyphenyl)-4-methylthiazol-2-yl)acetamide, with a structural formula as below:

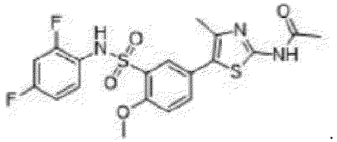

The starting material 1-(4-chlorophenyl)-2-acetone in Step 1 of Example 1 was replaced with 1-(4-methoxyphenyl)-2-acetone and the fragment tert-butyl (2-aminoethyl)carbamate in Step 2 of Example 1 was replaced with 2,4-difluorophenylamine, while other steps and procedures were identical to those in Example 1. A white solid, yield: 39%. ¹H NMR (400 MHz, DMSO-*d₆*) *δ* 12.12 (d,*J* = 5.6 Hz, 1H), 9.89 (s, 1H), 7.81-7.53 (m, 2H), 7.37-7.16 (m, 3H), 7.11-6.87 (m, 1H), 3.90 (d,*J* = 22.1 Hz, 4H), 2.25-2.10 (m, 6H); ¹³C NMR (101 MHz, DMSO) *δ* 168.40, 155.64, 155.01, 142.09, 134.86, 129.78, 129.08, 128.91, 128.86, 128.80, 128.77, 127.48, 123.76, 122.01, 113.61, 111.63, 111.58, 104.70, 104.41, 104.17, 56.26, 22.43, 14.22; ESI-HRMS calcd for C₁₉H₁₇F₂N₃O₄S₂ *m*/*z* [M+H]⁺ 454.0701, found [M+H]⁺ 454.0709.

### Example 8

Tert-butyl (5-(2-propionamido-4-methylthiazol-5-yl)-2-methoxyphenylsulfonamido)ethyl carbamate, with a structural formula as below:

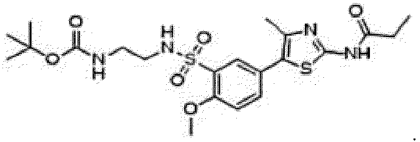

The starting material 1-(4-chlorophenyl)-2-acetone in Step 1 of Example 1 was replaced with 1-(4-methoxyphenyl)-2-acetone, and acetyl chloride in Step 4 was replaced with propanoyl chloride, while other steps and procedures were identical to those in Example 1. A white solid, yield: 32%. ¹H NMR (400 MHz, DMSO-*d₆*) *δ* 12.09 (s, 1H), 7.72 (d,*J* = 2.4 Hz, 1H), 7.67 (dd,*J* = 8.5, 2.4 Hz, 1H), 7.38 (t,*J* = 5.9 Hz, 1H), 7.31 (d,*J* = 8.7 Hz, 1H), 6.75 (t,*J* = 5.7 Hz, 1H), 3.94 (s, 3H), 2.96 (q,*J* = 6.5 Hz, 2H), 2.83 (q,*J* = 6.6 Hz, 2H), 2.43 (q,*J* = 7.5 Hz, 2H), 2.32 (s, 3H), 1.34 (s, 9H), 1.09 *(t,J=* 7.6 Hz, 3H); ¹³C NMR (101 MHz, DMSO-d₆) *δ* 172.07, 155.46, 155.30, 155.06, 142.07, 134.25, 128.90, 128.20, 124.06, 122.22, 113.63, 77.80, 56.37, 42.39, 28.23, 28.19, 15.84, 9.18; ESI-HRMS calcd for C₂₁H₃₀N₄O₆S₂ m/z [M+H]⁺ 499.1680, found [M+H]⁺ 499.1671.

### Example 9

Tert-butyl (5-(2-butanamido-4-methylthiazol-5-yl)-2-methoxyphenylsulfonamido)ethyl carbamate, with a structural formula as below:

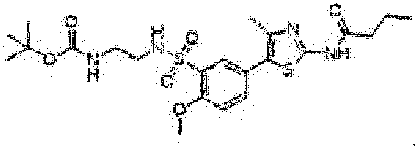

The starting material 1-(4-chlorophenyl)-2-acetone in Step 1 of Example 1 was replaced with 1-(4-methoxyphenyl)-2-acetone, and acetyl chloride in Step 4 was replaced with butanoyl chloride, while other steps and procedures were identical to those in Example 1. A white solid, yield: 34%. ¹H NMR (400 MHz, DMSO-d₆) *δ* 12.11 (s, 1H), *7.72* (d,*J =* 2.4 Hz, 1H), 7.67 (dd,*J* = 8.6, 2.4 Hz, 1H), 7.37 ( (t,*J =* 6.9, 6.4 Hz, 1H), 7.31 *(d,J =* 8.6 Hz, 1H), 6.74 (t,*J =* 5.6 Hz, 1H), 3.94 (s, 3H), 2.96 (q,*J* = 6.5 Hz, 2H), 2.88-2.76 (m, 2H), 2.40 (t,*J =* 7.3 Hz, 2H), 2.32 (s, 3H), 1.66-1.57 (m, 2H), 1.34 (s, 9H), 0.89 (t,J= 7.4 Hz, 3H); ¹³C NMR (101 MHz, DMSO-d₆) *δ* 171.24, 155.47, 155.31, 154.99, 142.07, 134.26, 128.93, 128.20, 124.06, 122.26, 113.62, 77.80, 56.37, 42.39, 36.78, 28.18, 18.23, 15.82, 13.50; ESI-HRMS calcd for C₂₂H₃₂N₄O₆S₂ m/z [M+H]⁺ 513.1836, found [M+H]⁺ 513.1841.

### Example 10

Tert-butyl (5-(2-pentanamido-4-methylthiazol-5-yl)-2-methoxyphenylsulfonamido)ethyl carbamate, with a structural formula as below:

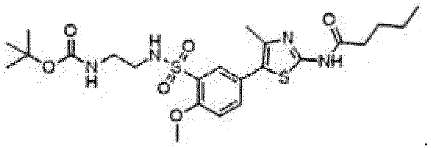

The starting material 1-(4-chlorophenyl)-2-acetone in Step 1 of Example 1 was replaced with 1-(4-methoxyphenyl)-2-acetone, and acetyl chloride in Step 4 was replaced with pentanoyl chloride, while other steps and procedures were identical to those in Example 1. A white solid, yield: 35%. ¹H NMR (300 MHz, DMSO-d₆) *δ* 12.12 (s, 1H), *7.72 (d,J =* 2.4 Hz, 1H), 7.68 (dd,*J* = 8.5, 2.4 Hz, 1H), 7.46 (t,*J* = 5.9 Hz, 2H), 7.31 (d,*J =* 8.7 Hz, 1H), 3.94 (s, 3H), 3.15-3.03 (m, 2H), 2.83 (q,*J* = 6.6 Hz, 2H), 2.42 (t,J= 7.4 Hz, 2H), 2.32 (s, 3H), 1.63-1.53 (m, 2H), 1.36-1.24 (m, 2H), 1.04 (s, 9H), 0.88 (t,J=7.3 Hz, 3H); ¹³C NMR (101 MHz, DMSO) *δ* 171.42, 155.50, 155.33, 155.02, 142.10, 134.29, 128.94, 128.23, 124.08, 122.29, 113.66, 77.83, 56.40, 42.41, 40.17, 39.96, 39.75, 39.54, 39.33, 39.12, 38.92, 34.88, 28.20, 24.45, 21.85, 15.83, 13.85; ESI-HRMS calcd for C₂₃H₃₄N₄O₆S₂ m/z [M+H]⁺ 527.1993, found [M+H]⁺ 527.1995.

### Example 11

Tert-butyl (5-(2-hexanamido-4-methylthiazol-5-yl)-2-methoxyphenylsulfonamido)ethyl carbamate, with a structural formula as below:

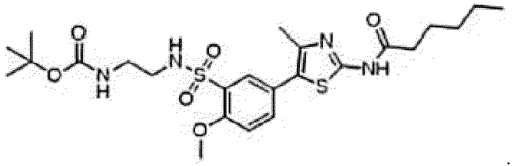

The starting material 1-(4-chlorophenyl)-2-acetone in Step 1 of Example 1 was replaced with 1-(4-methoxyphenyl)-2-acetone, and acetyl chloride in Step 4 was replaced with hexanoyl chloride, while other steps and procedures were identical to those in Example 1. A white solid, yield: 34%. ¹H NMR (300 MHz, DMSO-*d₆*) *δ* 12.11 (s, 1H), 7.72 (d,*J* = 2.4 Hz, 1H), 7.67 (dd,*J*) = 8.5, 2.4 Hz, 1H), 7.38 (t,*J* = 5.8 Hz, 1H), 7.31 (d,*J* = 8.7 Hz, 1H), 6.74 (t,*J* = 5.5 Hz, 1H), 3.94 (s, 3H), 3.03-2.90 (m, 2H), 2.90-2.76 (m, 2H), 2.41 (t,*J* = 7.3 Hz, 2H), 2.31 (s, 3H), 1.64-1.54 (m, 2H), 1.34 (s, 9H). 1.29-1.22 (m, 4H), 0.88-0.84 (m, 3H); ¹³C NMR (101 MHz, DMSO-*d₆*) *δ* 171.40, 155.48, 155.31, 155.00, 142.08, 134.27, 128.92, 128.21, 124.06, 122.27, 113.64, 77.81, 56.38, 42.39, 34.86, 30.72, 28.18, 24.43, 21.83, 15.81, 13.83; ESI-HRMS calcd for C₂₄H₃₆N₄O₆S₂ *m*/*z* [M+H]⁺ 541.2149, found [M+H]⁺ 541.2147.

### Example 12

Tert-butyl (5-(2-heptanamido-4-methylthiazol-5-yl)-2-methoxyphenylsulfonamido)ethyl carbamate, with a structural formula as below:

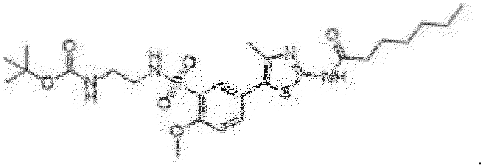

The starting material 1-(4-chlorophenyl)-2-acetone in Step 1 of Example 1 was replaced with 1-(4-methoxyphenyl)-2-acetone, and acetyl chloride in Step 4 was replaced with heptanoyl chloride, while other steps and procedures were identical to those in Example 1; a white solid, yield: 35%. ¹H NMR (400 MHz, DMSO-*d₆*) *δ* 12.11 (s, 1H), 7.72 (d,*J* = 2.4 Hz, 1H), 7.67 (dd,*J* = 8.6, 2.4 Hz, 1H), 7.38 (t,*J* = 5.8 Hz, 1H), 7.31 (d,*J* = 8.6 Hz, 1H), 6.74 (t,*J* = 5.7 Hz, 1H), 3.94 (s, 3H), 2.95 (q,*J* = 6.5 Hz, 2H), 2.86-2.77 (m, 2H), 2.41 (t,*J* = 7.4 Hz, 2H), 2.32 (s, 3H), 1.62-1.55 (m, 2H), 1.34 (s, 9H), 1.30-1.26 (m, 6H), 0.87-0.84 (m, 3H); ¹³C NMR (101 MHz, DMSO-*d₆*) *δ* 171.36, 155.45, 155.29, 154.98, 142.06, 134.24, 128.91, 128.20, 124.04, 122.25, 113.62, 77.78, 56.35, 42.37, 34.88, 30.93, 28.17, 24.68, 21.95, 15.80, 13.91; ESI-HRMS calcd for C₂₅H₃₈N₄O₆S₂ *m*/*z* [M+H]⁺ 555.2306, found [M+H]⁺ 555.2310.

### Example 13

Tert-butyl (2-(2-methoxy-5-(4-methyl-2-(3-propylureido)thiazol-5-yl)phenyl)sulfonamido)ethyl carbamate, with a structural formula as below:

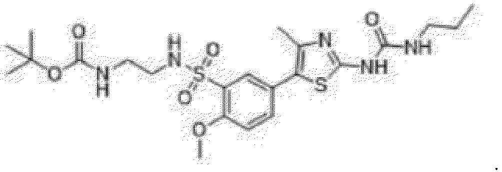

The starting material 1-(4-chlorophenyl)-2-acetone in Step 1 of Example 1 was replaced with 1-(4-methoxyphenyl)-2-acetone, and acetyl chloride in Step 4 was replaced with propylcarbamoyl chloride, while other steps and procedures were identical to those in Example 1. A white solid, yield: 36%. ¹H NMR (400 MHz, DMSO-*d₆*) *δ* 10.36 (s, 1H), 7.68 (d,*J* = 2.4 Hz, 1H), 7.63 (dd,*J* = 8.6, 2.4 Hz, 1H), 7.39 (t,*J* = 5.9 Hz, 1H), 7.29 (d,*J* = 8.7 Hz, 1H), 6.76 (t,*J* = 5.7 Hz, 1H), 6.57 (t,*J* = 5.9 Hz, 1H), 3.93 (s, 3H), 3.08 (q, *J* = 6.6 Hz, 2H), 2.95 (q,*J* = 6.5 Hz, 2H), 2.81 (q,*J* = 6.6 Hz, 2H), 2.26 (s, 3H), 1.49-1.40 (m, 2H), 1.33 (s, 9H), 0.86 (t,*J* = 7.4 Hz, 3H); ¹³C NMR (101 MHz, DMSO-*d₆*) *δ* 156.83, 155.50, 155.05, 152.76, 141.76, 134.00, 128.72, 128.09, 124.51, 120.77, 113.60, 77.85, 65.01, 56.37, 50.02, 42.41, 28.81, 28.22, 15.96, 15.24; ESI-HRMS calcd for C₂₂H₃₃N₅O₆S₂ *m*/*z* [M+H]⁺ 528.1945, found [M+H]⁺ 528.1940.

### Example 14

Tert-butyl (2-(5-(2-(3-butylureido)-4-methylthiazol-5-yl)-2-methoxyphenylsulfonamido)ethyl) carbamate, with a structural formula as below:

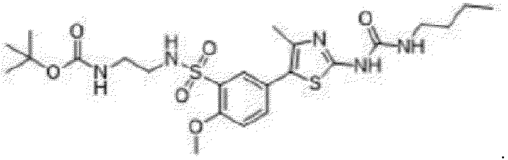

The starting material 1-(4-chlorophenyl)-2-acetone in Step 1 of Example 1 was replaced with 1-(4-methoxyphenyl)-2-acetone, and acetyl chloride in Step 4 was replaced with butylcarbamoyl chloride, while other steps and procedures were identical to those in Example 1. A white solid, yield: 42%. ¹H NMR (400 MHz, DMSO-*d₆*) *δ* 10.33 (s.1H), 7.69 (d,*J* = 2.4 Hz, 1H), 7.63 (dd,*J* = 8.6, 2.4 Hz, 1H), 7.36 (t,*J* = 5.8 Hz, 1H), 7.29 (d,*J* = 8.7 Hz, 1H), 6.74 (t,*J* = 5.7 Hz, 1H), 6.56-6.53 (m, 1H), 3.93 (s, 3H), 3.12 (q,*J* = 6.5 Hz, 2H), 2.95 (q,*J* = 6.5 Hz, 2H), 2.82 (q,*J* = 6.5 Hz, 2H), 2.26 (s, 3H), 1.46-1.39 (m, 2H), 1.34 (s, 9H), 1.29 (t,*J* = 7.5 Hz, 2H), 0.89 (t,*J* = 7.3 Hz, 3H); ¹³C NMR (101 MHz, DMSO-*d₆*) *δ* 164.06, 155.50, 155.08, 153.83, 141.81, 134.05, 128.73, 128.10, 124.45, 113.60, 77.85, 65.01, 56.37, 42.41, 41.05, 28.22, 22.82, 15.93, 15.24, 11.30; ESI-HRMS calcd for C₂₃H₃₅N₅O₆S₂ *m*/*z* [M+H]⁺ 542.2102, found [M+H]⁺ 542.2105.

### Example 15

Tert-butyl (2-(2-methoxy-5-(4-methyl-2-(3-methylbutanamido)thiazol-5-yl)phenyl)sulfonamido)ethyl carbamate, with a structural formula as below:

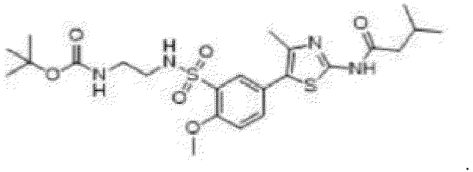

The starting material 1-(4-chlorophenyl)-2-acetone in Step 1 of Example 1 was replaced with 1-(4-methoxyphenyl)-2-acetone, and acetyl chloride in Step 4 was replaced with 3-methylbutanoyl chloride, while other steps and procedures were identical to those in Example 1. A white solid, yield: 40%. ¹H NMR (400 MHz, DMSO-*d₆*) *δ* 12.10 (s, 1H), 7.72 (d,*J* = 2.4 Hz, 1H), 7.68 (dd,*J* = 8.6, 2.4 Hz, 1H), 7.37 (t,*J* = 5.8 Hz, 1H), 7.31 (d,*J* = 8.7 Hz, 1H), 6.73 (t,*J* = 5.7 Hz, 1H), 3.94 (s, 3H), 2.96 (q,*J* = 6.5 Hz, 2H), 2.83 (q,*J* = 6.3 Hz, 2H), 2.31 (d,*J* = 7.7 Hz, 5H), 2.11-2.03 (m, 1H), 1.34 (s, 9H), 0.92 (d,*J* = 6.7 Hz, 6H); ¹³C NMR (75 MHz, DMSO-*d₆*) *δ* 170.76, 155.49, 155.34, 154.95, 142.11, 134.31, 128.98, 128.19, 124.07, 122.31, 113.64, 77.83, 59.81, 56.39, 43.94, 42.41, 28.20, 25.59, 22.19, 15.83; ESI-HRMS calcd for C₂₃H₃₄N₄O₆S₂ *m*/*z* [M+H]⁺ 527.1993, found [M+H]⁺ 527.2001.

### Example 16

Tert-butyl (5-(2-isobutanamido-4-methylthiazol-5-yl)-2-methoxyphenylsulfonamido)ethyl carbamate, with a structural formula as below:

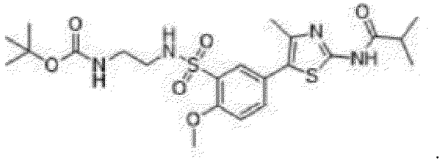

The starting material 1-(4-chlorophenyl)-2-acetone in Step 1 of Example 1 was replaced with 1-(4-methoxyphenyl)-2-acetone, and acetyl chloride in Step 4 was replaced with 2-methylpropanoyl chloride, while other steps and procedures were identical to those in Example 1. A white solid, yield: 26%. ¹H NMR (400 MHz, DMSO-*d₆*) *δ* 12.11 (s, 1H), 7.72 (d,*J* = 2.4 Hz, 1H), 7.67 (dd,*J* = 8.6, 2.4 Hz, 1H), 7.39 (t,*J* = 5.9 Hz, 1H), 7.31 (d,*J* = 8.7 Hz, 1H), 6.75 (t,*J* = 5.7 Hz, 1H), 3.94 (s, 3H), 2.95 (q.*J* = 6.6 Hz, 2H), 2.82 (q,*J* = 6.5 Hz, 2H), 2.75-2.69 (m, 1H), 2.32 (s, 3H), 1.34 (s, 9H), 1.11 (d,*J* = 6.9 Hz, 6H); ¹³C NMR (101 MHz, DMSO-*d₆*) *δ* 175.22, 155.47, 155, 32, 155.15, 142.09. 134.29, 128.93, 128.23, 124.06, 122.35, 113.64, 77.80, 56.38, 42.40, 39.82, 33.82, 28.19, 19.11, 15.80; ESI-HRMS calcd forC₂₂H₃₂N₄O₆S₂ m/z [M+H]⁺ 513.1836.found [M+H]⁺ 513.1839.

### Example 17

Tert-butyl (2-(2-methoxy-5-(4-methyl-2-(2,2-dimethylpropanamido)thiazol-5-yl)phenyl)sulfonamido)ethyl carbamate, with a structural formula as below:

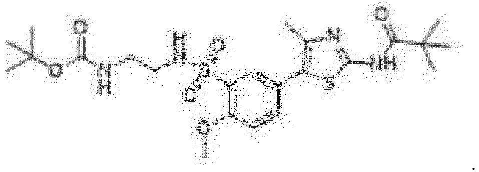

The starting material 1-(4-chlorophenyl)-2-acetone in Step 1 of Example 1 was replaced with 1-(4-methoxyphenyl)-2-acetone, and acetyl chloride in Step 4 was replaced with 2,2-dimethylpropanoyl chloride, while other steps and procedures were identical to those in Example 1; a white solid, yield: 30%. ¹H NMR (300 MHz, DMSO-d₆) *δ* 11.85 (s, 1H), 7.72 (d,*J =* 2.4 Hz, 1H), 7.67 (dd,*J* = 8.5, 2.4 Hz, 1H), 7.40 (t,J= 5.9 Hz, 1H), 7.32 (d,*J =* 8.6 Hz, 1H), 6.76 (t,*J =* 5.6 Hz, 1H), 3.94 (s, 3H), 3.05-2.90 (m, 2H), 2.83 (q,*J* = 6.8 Hz, 2H), 2.33 (s, 3H), 1.34 (s, 9H), 1.24 (s, 9H); ¹³C NMR (101 MHz, DMSO) *δ* 176, 70, 155.79, 155.48, 155.32, 141.99, 134.28, 128.95, 128.26, 124.11, 122.42, 113.64, 77.81, 56.37, 42.43, 38.77, 28.18, 26.60, 15.71; ESI-HRMS calcd for C₂₃H₃₄N₄O₆S₂ m/z [M+H]⁺ 527.1993, found [M+H]⁺ 527.2000.

### Example 18

Tert-butyl (5-(2-(3., 3-dimethylbutanamido)-4-methylthiazol-5-yl)-2-methoxyphenylsulfonamido)ethyl carbamate, with a structural formula as below:

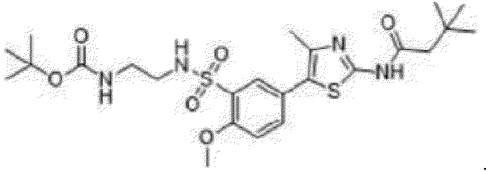

The starting material 1-(4-chlorophenyl)-2-acetone in Step 1 of Example 1 was replaced with 1-(4-methoxyphenyl)-2-acetone, and acetyl chloride in Step 4 was replaced with 3,3-dimethylbutanoyl chloride, while other steps and procedures were identical to those in Example 1. A white solid, yield: 40%. ¹H NMR (400 MHz, DMSO-d₆) *δ* 12.05 (s, 1H), 7.73 (d,J= 2.4 Hz, 1H), 7.68 (dd,*J* = 8.6, 2.4 Hz, 1H), 7.36 (t,J= 5.8 Hz, 1H), 7.31 (d,*J =* 8.6 Hz, 1H), 6.73 (t,*J =* 5.7 Hz, 1H), 3.94 (s, 3H), 2.95 (q,*J* = 6.5 Hz, 2H), 2.89-2.77 (m, 2H), 2.32 (d,*J =* 2.6 Hz, 5H), 1.34 (s, 9H), 1.00 (s, 9H); ¹³C NMR (101 MHz, DMSO-d₆) *δ* 174.22, 155.45, 155.29, 155.10, 142.05, 134.25, 128.90, 128.22, 124.07, 122.27, 113.62, 77.78, 56.35, 43.33, 42.38, 28.76, 28.17, 25.03, 15.78; ESI-HRMS calcd for C₂₄H₃₆N₄O₆S₂ m/z [M+H]⁺ 541.2149, found [M+H]⁺ 541.2152.

### Example 19

Tert-butyl (2-(2-methoxy-5-(4-methyl-2-(4-methylpentanamido)thiazol-5-yl)phenyl)sulfonamido)ethyl carbamate, with a structural formula as below:

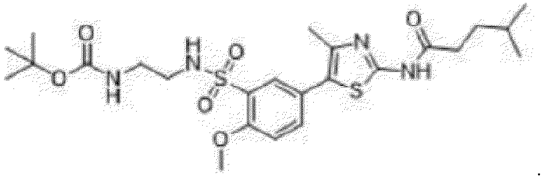

The starting material 1-(4-chlorophenyl)-2-acetone in Step 1 of Example 1 was replaced with 1-(4-methoxyphenyl)-2-acetone, and acetyl chloride in Step 4 was replaced with 4-methylpentanoyl chloride, while other steps and procedures were identical to those in Example 1. A white solid, yield: 34%. ¹H NMR (400 MHz, DMSO-d₆) *δ* 12.14 (s, 1H), 7.71 (d,*J =* 2.4 Hz, 1H), 7.67 (dd,*J* = 8.6, 2.4 Hz, 1H), 7.40 (t,*J =* 5.9 Hz, 1H), 7.31 (d,*J =* 8.6 Hz, 1H), 6.76 (t,*J =* 5.7 Hz, 1H), 3.94 (s, 3H), 2.95 (q,*J* = 6.5 Hz, 2H), 2.86-2.75 (m, 2H), 2.43 (t,*J =* 7.3 Hz, 2H), 2.31 (s, 3H), 1.53-1.47 (m, 3H), 1.33 (s, 9H), 0.87 (d,*J =* 6.2 Hz, 6H); ¹³C NMR (101 MHz, DMSO) *δ* 169.96, 155.44, 155.29, 154, 81, 142.05, 134.24, 128.93, 128.18, 124.05, 122.26, 113.60, 77.77, 56.35, 47.91, 42.37, 30.98, 29.44, 28.16, 15.79; ESI-HRMS calcd for C₂₄H₃₆N₄O₆S₂ m/z [M+H]⁺ 541.2149, found [M+H]⁺ 541.2143.

### Example 20

Tert-butyl (2-(2-methoxy-5-(4-methyl-2-(5-methylhexanamido)thiazol-5-yl)phenyl)sulfonamido)ethyl carbamate, with a structural formula as below:

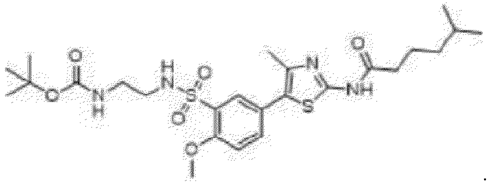

The starting material 1-(4-chlorophenyl)-2-acetone in Step 1 of Example 1 was replaced with 1-(4-methoxyphenyl)-2-acetone, and acetyl chloride in Step 4 was replaced with 5-methylhexanoyl chloride, while other steps and procedures were identical to those in Example 1. A white solid, yield: 34%. ¹H NMR (400 MHz, DMSO-d₆) *δ* 12.09 (s, 1H), 7.73 (t,J= 1.9 Hz, 1H), 7.67 (dt,J= 8.7, 2.1 Hz, 1H), 7.37 (t,J= 5.8 Hz, 1H), 7.31 (dd,*J* = 8.7, 1.8 Hz, 1H), 6.73 (t,J= 5.8 Hz, 1H), 3.94 (s, 3H), 2.96 (q,J= 6.6 Hz, 2H), 2.83 (q,*J* = 6.6 Hz, 2H), 2.45-2.36 (m, 2H), 2.32 (d,*J =* 1.7 Hz, 3H), 1.67-1.46 (m, 3H), 1.34 (s, 9H), 1.21-1.11 (m, 2H), 0.86 (dd,*J* = 6.7, 1.9 Hz, 6H); ¹³C NMR (101 MHz, DMSO-d₆) *δ* 171.37, 155.47, 155.31, 155.00, 142.07, 134.26, 128.93, 128.20, 124.06, 122.27, 113.62, 77.80, 56.37, 42.39, 37.81, 35.11, 28.18, 27.25, 22.66, 22.42, 15.82; ESI-HRMS calcd for C₂₅H₃₈N₄O₆S₂ m/z [M+H]⁺ 555.2306, found [M+H]⁺ 555.2307.

### Example 21

Tert-butyl (2-(5-(2-(cyclohexanecarboxamido)-4-methylthiazol-5-yl)-2-methoxyphenyl)sulfonamido)ethyl carbamate, with a structural formula as below:

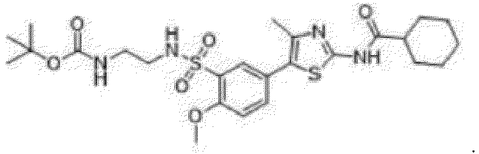

The starting material 1-(4-chlorophenyl)-2-acetone in Step 1 of Example 1 was replaced with 1-(4-methoxyphenyl)-2-acetone, and acetyl chloride in Step 4 was replaced with cyclohexanecarbonyl chloride, while other steps and procedures were identical to those in Example 1. A white solid, yield: 45%. ¹H NMR (400 MHz, DMSO-d₆) *δ* 12.06 (s, 1H), 7.71 (d,*J =* 2.4 Hz, 1H), 7.66 (dd,*J* = 8.6, 2.4 Hz, 1H), 7.38 (t,J= 5.9 Hz, 1H), 7.31 (d,*J =* 8.7 Hz, 1H), 6.74 (t,*J =* 5.8 Hz, 1H) , 3.94 (s, 3H), 2.95 (q,*J* = 6.5 Hz, 2H), 2.82 (q,J= 6.5 Hz, 2H), 2.45 (dt,J= 11.4, 3.5 Hz, 1H), 2.31 (s, 3H), 1.82-1.72 (m, 4H), 1.45-1.38 (m, 2H), 1.34 (s, 9H), 1.29-1.15 (m, 4H); ¹³C NMR (101 MHz, DMSO-d₆) *δ* 174.26, 155.48, 155.32, 155.12, 142.08, 134.29, 128.92, 128.21, 124.08, 122.29, 113.65, 77.82, 56.39, 43.36, 42.40, 39.82, 28.79, 28.20, 25.29, 25.06, 15.82; ESI-HRMS calcd for C₂₅H₃₆N₄O₆S₂ m/z [M+H]⁺ 553.2149, found [M+H]⁺ 553.2153.

### Example 22

Tert-butyl (2-(2-chloro-5-(4-methyl-2-(3-methylbutanamido)thiazol-5-yl)phenyl)sulfonamido)ethyl carbamate, with a structural formula as below:

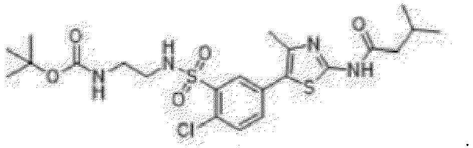

Acetyl chloride in Step 4 of Example 1 was replaced with 3-methylbutanoyl chloride, while other steps and procedures were identical to those in Example 1. A white solid, yield: 35%. ¹H NMR (300 M4 Hz, DMSO-*d₆*) *δ* 12.23 (s, 1H), 8.05 (t,*J* = 5.6 Hz, 1H), 7.97-7.91 (m, 1H), 7.72 (t,*J* = 1.4 Hz, 2H), 6.79 (t,*J* = 5.5 Hz, 1H), 2.97 (dd,*J* = 12.2, 6.6 Hz, 2H), 2.91 (d,*J* = 6.7 Hz, 2H), 2.38 (s, 3H), 1.34 (s, 8H), 1.16 (dd,*J* = 18.6, 7.0 Hz, 8H); ¹³C NMR (101 MHz, DMSO-*d₆*) *δ* 175.85, 156.46, 155.94, 144.22, 138.72, 133.64, 132.94, 132.27, 129.82, 129.38, 121.86, 78.28, 60.22, 42.65, 34.28, 28.62, 21.23, 19.52, 16.55, 14.55; ESI-HRMS calcd for C₂₂H₃₁N₄O₅S₂ *m*/*z* [M+H]⁺ 531.1497, found [M+H]⁺ 531.1495.

### Example 23

Tert-butyl (2-chloro-5-(2-(3,3-dimethylbutanamido)-4-methylthiazol-5-yl)phenylsulfonamido)ethyl carbamate, with a structural formula as below:

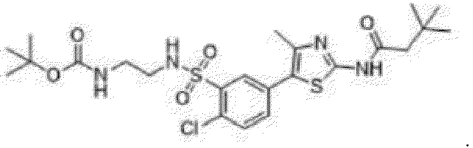

Acetyl chloride in Step 4 of Example 1 was replaced with 3,3-dimethylbutanoyl chloride, while other steps and procedures were identical to those in Example 1. A white solid, yield: 35%. ¹H NMR (300 MHz, DMSO-*d₆*) *δ* 12.19 (s, 1H), 8.04 (t,*J* = 5.7 Hz, 1H), 7.94 (t,*J* = 1.3 Hz, 1H), 7.72 (d,*J* = 1.3 Hz, 2H), 6.78 (t,*J* = 5.6 Hz, 1H), 2.98-2.88 (m, 4H), 2.37 (s, 3H), 2.33 (s, 2H), 1.33 (s, 9H), 1.00 (s, 9H); ¹³C NMR (101 MHz, DMSO-*d₆*) *δ* 170.17, 155.67, 155.48, 143.75, 138.21, 133.18, 132.45, 131.82, 129.40, 128.90, 121.34, 77.81, 47.90, 42.17, 40.15, 39.94, 39.73, 39.52, 39.31, 39.10, 38.89, 31.01, 29.43, 28.16, 16.09; ESI-HRMS calcd for C₂₃H₃₃CIN₄O₆S₂ *m*/*z* [M+H]⁺ 545.1654, found [M+H]⁺ 545.1660.

### Example 24

Tert-butyl (2-chloro-5-(4-methyl-2-pentanamido-5-yl)phenylsulfonamido)ethyl carbamate, with a structural formula as below:

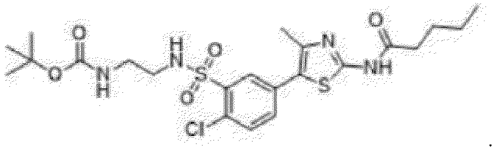

Acetyl chloride in Step 4 of Example 1 was replaced with pentanoyl chloride, while other steps and procedures were identical to those in Example 1. A white solid, yield: 35%. ¹H NMR (400 MHz, DMSO-*d₆*) *δ* 12.22 (s, 1H), 8.03 (t,*J* = 5.6 Hz, 1H), 7.93 (s, 1H), 7.73-7.69 (m, 2H), 6.77 (t,*J* = 5.6 Hz, 1H), 2.98 (q,*J* = 6.5 Hz, 2H), 2.90 (q,*J* = 6.8, 6.2 Hz, 2H), 2.43 (t,*J* = 7.4 Hz, 2H), 2.37 (s, 3H), 1.62-1.54 (m, 2H), 1.33 (s, 9H), 1.31-1.25 (m, 2H), 0.88 (t,*J* = 7.4 Hz, 3H); ¹³C NMR (101 MHz, DMSO-*d₆*) *δ* 171.59, 155.87, 155.50, 143.77, 138.25, 133.19, 132.48, 131.82, 129.39, 128.92, 121.34, 77.84, 42.20, 34.61, 28.17, 26.81, 21.67, 16.11, 13.65; ESI-HRMS calcd forC₂₂H₃₁CIN₄O₅S₂ *m*/*z* [M+H]⁺ 531.1497, found [M+H]⁺ 531.1498.

### Example 25

Tert-butyl (2-chloro-5-(2-hexanamido-4-methylthiazol-5-yl)phenylsulfonamido)ethyl carbamate, with a structural formula as below:

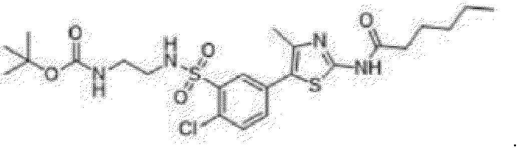

Acetyl chloride in Step 4 of Example 1 was replaced with hexanoyl chloride, while other steps and procedures were identical to those in Example 1. A white solid, yield: 36%. ¹H NMR (400 MHz, DMSO-*d₆*) *δ* 12.20 (s, 1H), 8.02 (s, 1H), 7.93 (s, 1H), 7.71 (d,*J* = 1.8 Hz, 2H), 6.85-6.65 (m, 1H), 2.98 (q,*J* = 6.5 Hz, 2H), 2.91 (q,*J* = 6.6 Hz, 2H), 2.43 (t,*J* = 7.4 Hz, 2H), 2.37 (s, 3H), 1.64-1.56 (m, 2H), 1.33 (s, 9H), 1.30-1.24 (m, 4H), 0.86 (t,*J* = 6.8 Hz, 3H); ¹³C NMR (101 MHz, DMSO-*d₆*) *δ* 171.56, 155.84, 155.47, 143.74, 138.24, 133.16, 132.46, 131.81, 129.37, 128.90, 121.32, 77.81, 42.18, 39.87, 34.84, 30.69, 28.15, 24.37, 21.80, 16.10, 13.81; ESI-HRMS calcd for C₂₃H₃₃CIN₄O₅S₂ *m*/*z* [M+H]⁺ 545.1654, found [M+H]⁺ 545.1645.

### Example 26

Tert-butyl (2-(5-(2-(3-acetamidopropionamido)-4-methylthiazol-5-yl)-2-methoxyphenyl)sulfonamido)ethyl carbamate, with a structural formula as below:

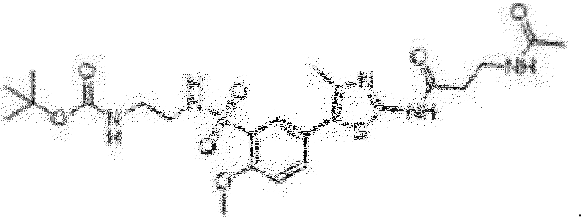

The starting material 1-(4-chlorophenyl)-2-acetone in Step 1 of Example 1 was replaced with 1-(4-methoxyphenyl)-2-acetone, and acetyl chloride in Step 4 was replaced with 3-acetamidopropanoyl chloride, while other steps and procedures were identical to those in Example 1. A white solid, yield: 30%. ¹H NMR (300 MHz, DMSO-*d₆*) *δ* 12.17 (s, 1H), 7.99 (t,*J* = 5.7 Hz, 1H), 7.72 (d,*J* = 2.4 Hz, 1H), 7.68 (dd,*J* = 8.5, 2.4 Hz, 1H), 7.39 (t,*J* = 5.8 Hz, 1H), 7.31 (d,*J* = 8.7 Hz, 1H), 6.83-6.67 (m, 1H), 3.94 (s, 3H), 3.31 (t,*J* = 6.2 Hz, 2H), 2.95 (q,*J* = 6.5 Hz, 2H), 2.82 (q,*J* = 6.4 Hz, 2H), 2.59 (t,*J* = 6.7 Hz, 2H), 2.32 (s, 3H), 1.78 (s, 3H), 1.34 (s, 9H); ¹³C NMR (101 MHz, DMSO-*d₆*) *δ* 169.72, 169.36, 155.50, 155.36, 154.91, 142.14, 134.33, 128.97, 128.20, 124.02, 122.36, 113.68, 77.86, 56.42, 42.40, 39.83, 35.16, 34.66, 28.22, 22.60, 15.88; ESI-HRMS calcd for C₂₃H₃₃N₅O₇S₂ *m*/*z* [M+H]⁺ 556.1894, found [M+H]⁺ 556.1896.

### Example 27

Tert-butyl (2-(2-methoxy-5-(4-methyl-2-(6-oxoheptanamido)thiazol-5-yl)phenyl)sulfonamido)ethyl carbamate, with a structural formula as below:

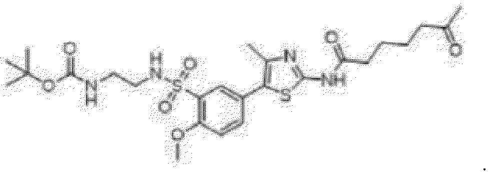

The starting material 1-(4-chlorophenyl)-2-acetone in Step 1 of Example 1 was replaced with 1-(4-methoxyphenyl)-2-acetone, and acetyl chloride in Step 4 was replaced with 6-oxoheptanoyl chloride, while other steps and procedures were identical to those in Example 1. A white solid, yield: 35%. ¹H NMR (400 MHz, DMSO-*d₆*) *δ* 12.10 (s, 1H), 7.72 (d,*J* = 2.4 Hz, 1H), 7.67 (dd,*J* = 8.5, 2.4 Hz, 1H), 7.36 (t,*J* = 5.8 Hz, 1H), 7.31 (d,*J* = 8.6 Hz, 1H), 6.73 (t,*J* = 5.7 Hz, 1H), 3.94 (s, 3H), 2.96 (q,*J* = 6.5 Hz, 2H), 2.89-2.77 (m, 2H), 2.43 (dt,*J* = 13.9, 7.3 Hz, 4H), 2.32 (s, 3H), 2.07 (s, 3H), 1.63-1.41 (m, 4H), 1.34 (s, 9H); ¹³C NMR (101 MHz, DMSO-*d₆*) *δ* 208.42, 171.28, 155.53, 155.36, 155.00, 142.13, 134.32, 128.96, 128.22, 124.07, 122.33, 113.67, 77.88, 56.41, 42.43, 42.36, 39.84, 34.77, 29.76, 28.21, 24.29, 22.71, 15.84; ESI-HRMS calcd for C₂₅H₃₆N₄O₇S₂ m/z [M+H]⁺ 569.2098, found [M+H]⁺ 569.2103.

### Example 28

Tert-butyl (2-(2-methoxy-5-(4-methyl-2-(6-oxohexanamido)thiazol-5-yl)phenyl)sulfonamido)ethyl carbamate, with a structural formula as below:

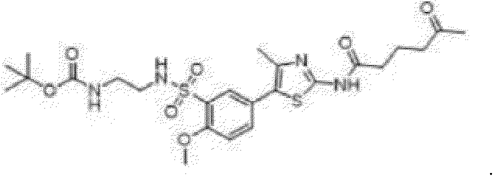

The starting material 1-(4-chlorophenyl)-2-acetone in Step 1 of Example 1 was replaced with 1-(4-methoxyphenyl)-2-acetone, and acetyl chloride in Step 4 was replaced with 5-oxohexanoyl chloride, while other steps and procedures were identical to those in Example 1. A white solid, yield: 35%. ¹H NMR (400 MHz, DMSO-d₆) *δ* 12.11 (s, 1H), *7.72* (d,*J =* 2.4 Hz, 1H), 7.67 (dd,*J* = 8.6, 2.4 Hz, 1H), 7.36 (t,*J =* 5.9 Hz, 1H), 7.31 (d,*J =* 8.7 Hz, 1H), 6.73 (t,*J* = 5.7 Hz, 1H), 3.94 (s, 3H), 2.96 (q,*J* = 6.5 Hz, 2H), 2.83 (q,*J* = 6.6 Hz, 2H), 2.46 (t,J= 3.6 Hz, 2H), 2.42 (t,*J =* 7.3 Hz, 2H), 2.32 (s, 3H), 2.08 (s, 3H), 1.77 (p,*J =* 7.4 Hz, 2H), 1.34 (s, 9H); ¹³C NMR (101 MHz, DMSO-d₆) *δ* 208.03, 171.04, 155.50, 155.34, 154.98, 142.10, 134.28, 128.96, 128, 23, 124.07, 122.32, 113.65, 77.84, 56.39, 41.86, 41.81, 34.01, 29.75, 28.20, 18.80, 15.83; ESI-HRMS calcd for C₂₄H₃₄N₄O₇S₂ m/z [M+H]⁺ 555.1942, found [M+H]⁺ 555.1946.

### Example 29

Tert-butyl (R)-(1-((5-(2-hexanamido-4-methylthiazol-5-yl)-2-methoxyphenyl)sulfonyl)piperidin-3-yl)carbamate, with a structural formula as below:

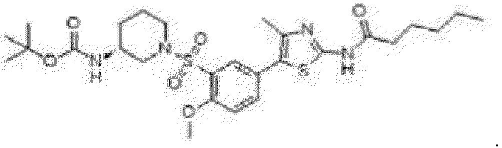

The starting material 1-(4-chlorophenyl)-2-acetone in Step 1 of Example 1 was replaced with 1-(4-methoxyphenyl)-2-acetone, the fragment tert-butyl (2-aminoethyl)carbamate in Step 2 was replaced with tert-butyl (R)-piperidin-3-yl carbamate, and acetyl chloride in Step 4 was replaced with hexanoyl chloride, while other steps and procedures were identical to those in Example 1. A white solid, yield: 18%. ¹H NMR (400 MHz, DMSO-d₆) *δ* 12.09 (s, 1H), 7.73 (d,J= 2.3 Hz, 1H), 7.69 (dd,*J* = 8.6, 2.5 Hz, 1H). 7.33 (d,*J =* 8.6 Hz, 1H), 6.83 (d,*J* = 7.7 Hz, 1H), 3.93 (s, 3H), 3.55 (dd,J = 63.9, 12.2 Hz, 2H), 2.59 *(t,J=* 11.5 Hz, 1H), 2.41 (t,J= 7.4 Hz, 2H), 2.31 (s, 3H), 1.76-1.68 (m.2H), 1.63-1.56 (m, 2H), 1.37 (s, 9H), 1.29 - 1.17 (m, 8H), 0.86 (t,*J* = 68 Hz, 3H); ¹³C NMR (101 MHz, DMSO-d₆) *δ* 171.43, 155.65, 155.06, 154.87, 142.17, 134.71, 130.23, 126.27, 124.24, 122.14, 113.99, 77.96, 56.34, 50.00, 46.74, 45.50, 34.87, 30.74, 29.42, 28.23, 24.45, 23.70, 21.86, 15.83, 13.86; ESI-HRMS calcd for C₂₇H₄₀N₄O₆S₂ m/z [M+H]⁺ 581.2462, found [M+H]⁺ 581.2471.

### Example 30

Tert-butyl (S)-(1-((5-(2-hexanamido-4-methylthiazol-5-yl)-2-methoxyphenyl)sulfonyl)pyrrolidin-3-yl)carbamate, with a structural formula as below:

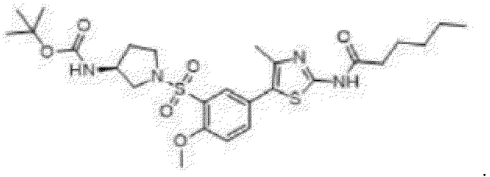

The starting material 1-(4-chlorophenyl)-2-acetone in Step 1 of Example 1 was replaced with 1-(4-methoxyphenyl)-2-acetone, the fragment tert-butyl (2-aminoethyl)carbamate in Step 2 was replaced with tert-butyl (S)-pyrrolidin-3-yl carbamate, and acetyl chloride in Step 4 was replaced with hexanoyl chloride, while other steps and procedures were identical to those in Example 1. A white solid, yield: 20%. ¹H NMR (300 MHz, DMSO-d₆) *δ* 12.11 (s, 1H), 7.76 (d,*J =* 2.4 Hz, 1H), 7.69 (dd,J = 8.6, 2.4 Hz, 1H), 7.34 (d,*J =* 8, 7 Hz, 1H), 7.11 (d,*J =* 6.5 Hz, 1H), 3.95 (s, 3H), 3.55-3.38 (m.2H), 3.25 (q,*J* = 7.9 Hz, 1H), 2.41 (t,J= 7.4 Hz, 2H), 2.32 (s, 3H), 2.06-1.66 (m, 2H), 1.65-1.53 (m, 2H), 1.35 (s, 9H), 1.34 -1.20 (m, 6H), 0.89-0.82 (m, 3H); ¹³C NMR (101 MHz, DMSO-d₆) *δ* 171.39, 155.59, 155.21, 155.02, 142.11, 134.55, 130.65, 126.31, 124.17, 122.18, 113.83, 78.02, 56.36, 52.49, 49.98, 45.99, 34.85, 30.73, 30.56, 28.17, 24.43, 21.85, 15.82, 13.84; ESI-HRMS calcd for C₂₆H₃₈N₄O₆S₂ m/z [M+H]⁺ 567.2306, found [M+H]⁺ 567.2313.

### Example 31

Tert-butyl 4-((2-hexanamido-4-methylthiazol-5-yl)-2-methoxyphenylsulfonamido)piperidin-1-carboxylate, with a structural formula as below:

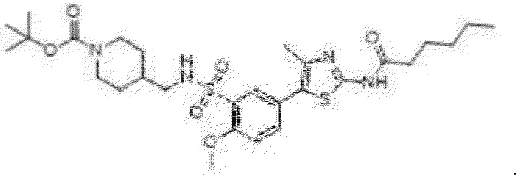

The starting material 1-(4-chlorophenyl)-2-acetone in Step 1 of Example 1 was replaced with 1-(4-methoxyphenyl)-2-acetone, the fragment tert-butyl (2-aminoethyl)carbamate in Step 2 was replaced with 4-aminomethylpiperidin-1-tert-butyl carboxylate, and acetyl chloride in Step 4 was replaced with hexanoyl hydrogen, while other steps and procedures were identical to those in Example 1. A white solid, yield: 40%. ¹H NMR (300 MHz, DMSO-d₆) *δ* 12.11 (s, 1H), 7.72 (d,J = 2.4 Hz, 1H), 7.67 (dd,*J* = 8.5, 2.4 Hz, 1H). 7.47 (t,*J =* 6.0 Hz, 1H), 7.30 (d,*J =* 8.6 Hz, 1H), 3.92 (s, 3H), 3.87 (t,*J =* 13.1 Hz, 2H), 2.69 (t,J= 6.3 Hz, 2H), 2.41 (t,J= 7.3 Hz, 2H), 2.31 (s, 3H), 1.64-1.49 (m, 5H), 1.36 (s, 9H). 1.28-1.14 (m, 6H), 0.96-0.83 (m, 5H); ¹³C NMR (101 MHz, DMSO-d₆) *δ* 171.46, 155.37, 155.02, 153.88, 142.07, 134.17, 129.00, 128.61, 124.03, 122.34, 113.61, 78.52, 59.82, 56.35, 47.98, 35.82, 34.89, 30.75, 28.12, 24.46, 21.86, 15.83, 13.86; ESI-HRMS calcd for C₂₈H₄₂N₄O₆S₂ m/z [M+H]⁺ 595.2619, found [M+H]⁺ 595.2624.

### Example 32

N-(5-(3-(2-(3,3-dimethylbutanamido)ethylaminosulfonyl)-4-methoxyphenyl)-4-methylthiazol-2-yl)hexanamide, with a structural formula as below:

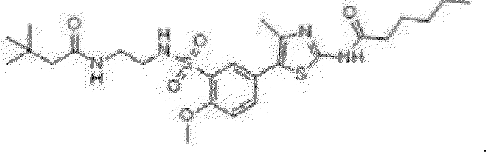

The starting material 1-(4-chlorophenyl)-2-acetone in Step 1 of Example 1 was replaced with 1-(4-methoxyphenyl)-2-acetone, the fragment tert-butyl (2-aminoethyl)carbamate in Step 2 was replaced with N-(2-aminoethyl)-3,3-dimethylbutanamide, and acetyl chloride in Step 4 was replaced with hexanoyl chloride, while other steps and procedures were identical to those in Example 1. A white solid, yield: 30%. ¹H NMR (300 MHz, DMSO-d₆) *δ* 12.11 (s, 1H), 7.84-7.59 (m, 3H), 7.42 (t,J= 5.9 Hz, 1H), 7.31 (d,J= 8.7 Hz, 1H), 3.93 (s, 3H), 3.37 (s, 3H), 3.07 (t,J= 6.6 Hz, 2H), 2.84 (q,*J* = 6.7 Hz, 2H), 2.41 (t,*J* = 7.4 Hz, 2H), 2.31 (s, 3H), 1.88 (s, 2H), 1.59 (t,*J =* 7.2 Hz, 2H), 1.32-1.21 (m, 4H), 0.97- 0.83 (m, 9H); ¹³C NMR (101 MHz, DMSO-d₆) *δ* 171.39, 171.06, 155.33, 155.02, 142.07, 134.25, 128.94, 128.23, 124.09, 122.26, 113.64, 56.38, 48.83, 42.40, 38.46, 34.87, 30.73, 30.29, 29.64, 24.44, 21.84, 15.81, 13.82; ESI-HRMS calcd for C₂₅H₃₈N₄O₅S₂ m/z [M+H]⁺ 539.2356, found [M+H]⁺ 539.2358.

### Example 33

N-(5-(3-(2-hydroxy-2-methylpropyl)aminosulfonyl)-4-methoxyphenyl)-4-methylthiazol-2-yl hexanamide, with a structural formula as below:

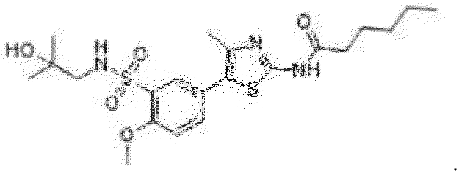

The starting material 1-(4-chlorophenyl)-2-acetone in Step 1 of Example 1 was replaced with 1-(4-methoxyphenyl)-2-acetone, the fragment tert-butyl (2-aminoethyl)carbamate in Step 2 was replaced with 1-amino-2-methyl-2-propanol, and acetyl chloride in Step 4 was replaced with hexanoyl chloride, while other steps and procedures were identical to those in Example 1. A white solid, yield: 31%. ¹H NMR (400 MHz, DMSO-d₆) *δ* 12.12 (s, 1H), 7.72 (d,J= 2.4 Hz, 1H), 7.68 (dd,J = 8.6, 2.4 Hz, 1H), 7.32 (d,J = 8.7 Hz, 1H), 6.96 (t,*J =* 6.4 Hz, 1H), 3.94 (s, 3H), 2.70 (d,J = 6.4 Hz, 2H), 2.41 (t,*J =* 7.4 Hz, 2H), 2.32 (s, 3H), 1.59 (p,*J =* 7.4 Hz, 2H), 1.32 - 1.23 (m, 4H), 1.06 (s, 6H), 0.92-0.81 (m, 3H); ¹³C NMR (101 MHz, DMSO-d₆) *δ* 171.37, 155.23, 154.97, 142.04, 134.12, 128.94, 128.23, 124.05, 122.25, 113.60, 68.74, 56.39, 53.89, 34.84, 30.71, 27.06, 24.40, 21.81, 15.81, 13.81; ESI-HRMS calcd for C₂₁H₃₀N₃O₅S₂ m/z [M+H]⁺ 470.1778, found [M+H]⁺ 470.1785.

### Example 34

N-(5-(3-(N-(2-morpholinoethyl)aminosulfonyl))-4-methoxyphenyl)-4-methylthiazol-2-yl hexanamide, with a structural formula as below:

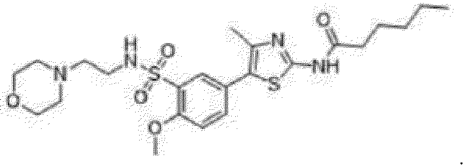

The starting material 1-(4-chlorophenyl)-2-acetone in Step 1 of Example 1 was replaced with 1-(4-methoxyphenyl)-2-acetone, and the fragment tert-butyl (2-aminoethyl)carbamate in Step 2 of Example 1 was replaced with N-(2-aminoethyl)morpholine, and acetyl chloride in Step 4 was replaced with hexanoyl chloride, while other steps and procedures were identical to those in Example 1. A white solid, yield: 20%. ¹H NMR (400 MHz, DMSO-d₆) *δ* 12.12 (s, 1H), 7.73 (d,*J =* 2.3 Hz, 1H), 7.68 (dd,*J* = 8.6, 2.4 Hz, 1H), 7.32 (d,*J =* 8.7 Hz, 1H), 7.16-7.04 (mn, 1H), 3.96 (s, 3H), 3.45 (t,*J =* 4.7 Hz, 4H), 2.96 (q,*J* = 6.3 Hz, 2H), 2.42 (t,J= 7.4 Hz, 2H), 2.32 (s, 3H), 2.28-2.19 (m, 6H), 1.79-1.73 (m, 2H), 1.37-1.17 (m, 4H), 0.90-0.85 (m, 3H); ¹³C NMR (101 MHz, DMSO) *δ* 155.35, 154.96, 136.08, 134.65, 130.56, 129.14, 126.96, 126.53, 113.86, 112.90, 63.17, 56.35, 55.51, 51.35, 47.92, 36.94, 30.70, 24.40, 21.80, 15.85, 13.82. ESI-HRMS calcd for C₂₃H₃₄N₄O₅S₂ m/z [M+H]⁺ 511.2043, found [M+H]⁺ 511.2044.

### Example 35

N-(5-(3-(((4-fluorophenyl)-12-nitridoacyl)sulfonyl)-4-methoxyphenyl)-4-methylthiazol-2-yl)hexanamide, with a structural formula as below:

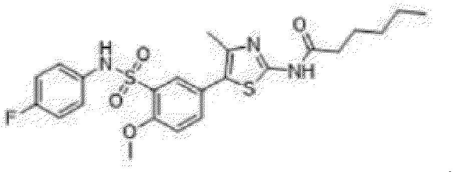

The starting material 1-(4-chlorophenyl)-2-acetone in Step 1 of Example 1 was replaced with 1-(4-methoxyphenyl)-2-acetone, the fragment tert-butyl (2-aminoethyl)carbamate in Step 2 was replaced with 4-fluorophenylamine, and acetyl chloride in Step 4 was replaced with hexanoyl chloride, while other steps and procedures were identical to those in Example 1; a white solid , yield: 55%. ¹H NMR (300 MHz, DMSO-d₆) *δ* 12.10 (s, 1H), 10.10 (s, 1H), 7.71-7.67 (m, 1H), 7.65-7.59 (m, 1H), 7.24 (dd,J= 9.1, 3.3 Hz, 1H), 7.15-7.01 (m, 4H), 3.91 *(d,J=* 2.0 Hz, 3H), 2.46-2.35 (m, 2H), 2.22 (q,*J* = 2.7, 1.9 Hz, 3H), 1.57 (d,*J =* 7.4 Hz, 2H), 1.30-1.19 (m, 4H), 0.88-0.82 (m, 3H); ¹³C NMR (101 MHz, DMSO-d₆) *δ* 171.47, 160.10 (d,*J =* 241.4 Hz), 155.43, 155.05, 142.21, 134.97, 134.01, 129.75, 126.46, 124.04, 122.19 (d,*J* = 8.1 Hz), 122.00, 115.91 (d,J= 23.2 Hz), 113.70, 56.42, 34.87, 30.75, 24.46, 21.87, 15.72, 13.87; ESI-HRMS calcd for C₂₃H₂₅FN₃O₄S₂ m/z [M+H]⁺ 492.1422, found [M+H]⁺ 492.1420.

### Example 36

N-(5-(3-(((4-chlorophenyl)-12-nitren)sulfonyl)-4-methoxyphenyl)-4-methylthiazol-2-yl)hexanamide, with a structural formula as below:

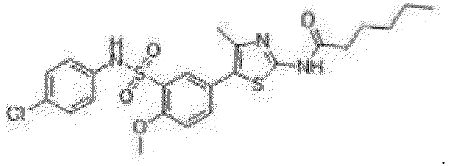

The starting material 1-(4-chlorophenyl)-2-acetone in Step 1 of Example 1 was replaced with 1-(4-methoxyphenyl)-2-acetone, the fragment tert-butyl (2-aminoethyl)carbamate in Step 2 was replaced with 4-chlorophenylamine, and acetyl chloride in Step 4 was replaced with hexanoyl chloride, while other steps and procedures were identical to those in Example 1; a white solid , yield: 49%. ¹H NMR (300 MHz, DMSO-d₆) *δ* 12.11 (s, 1H), 10.32 (s, 1H), 7.74 (d,*J =* 2.4 Hz, 1H), 7.63 (dd,*J* = 8.6, 2.4 Hz, 1H), 7.29-7.22 (m, 3H), 7.15-7.10 (m, 2H), 3.89 (s, 3H), 2.40 (t,J= 7.4 Hz, 2H), 2.24 (s, 3H), 1.58 (p,*J =* 7.3 Hz, 2H), 1.37-1.12 (m, 4H), 0.84 (t,J= 6.8 Hz, 3H); ¹³C NMR (101 MHz, DMSO-*d₆*) *δ* 171.45, 155.43, 155.09, 142.25, 136.83, 135.06, 129.79, 129.03, 127.86, 126.45, 124.12, 121.98, 121.06, 113.77, 56.44, 34.88, 30.75, 24.45, 21.86, 15.72, 13.85; ESI-HRMS calcd for C₂₃H₂₅CIN₃O₄S₂ m/z [M+H]⁺ 508.1126, found [M+H]⁺ 508.1133.

### Example 37

N-(5-(3-(((4-fluorobenzyl)-12-nitren)sulfonyl)-4-methoxyphenyl)-4-methylthiazol-2-yl)hexanamide, with a structural formula as below:

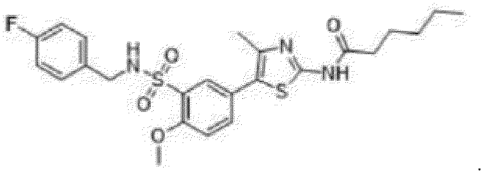

The starting material 1-(4-chlorophenyl)-2-acetone in Step 1 of Example 1 was replaced with 1-(4-methoxyphenyl)-2-acetone, and the fragment tert-butyl (2-aminoethyl)carbamate in Step 2 of Example 1 was replaced with 4-fluorobenzylamine, and acetyl chloride in Step 4 was replaced with hexanoyl chloride, while other steps and procedures were identical to those in Example 1; a white solid , yield: 54%. ¹H NMR (300 MHz, DMSO-d₆) *δ* 12.11 (s, 1H), 7.96 (t,J = 6.4 Hz, 1H), 7.64-7.52 (m, 2H), 7.23-7.09 (m, 3H), 6.96 (dd,J= 10.1.7.7 Hz, 2H), 4.08 *(d,J=* 6.4 Hz, 2H), 3.85 (s, 3H), 2.41 (t,J= 7.4 Hz, 2H), 2.29 (s, 3H), 1.65-1.54 (m, 2H), 1.26 (dd,*J* = 6.4, 2.9 Hz, 4H), 0.86 (t,J= 6.7 Hz, 3H); ¹³C NMR (101 MHz, DMSO-d₆) *δ* 171.41, 162.40 (d,*J* = 244.4 Hz). 155.11, 155.00, 141.96, 134.01, 133.71, 133.68, 129.71 (d,J= 8.1 Hz), 128.84, 123.78, 122.38, 114.68 (d,J= 21.2 Hz), 113.18, 56.15, 45.63, 34.88, 30.74, 24.45, 21.85, 15.72, 13.85; ESI-HRMS calcd for C₂₄H₂₈FN₃O₄S₂ m/z [M+H]⁺ 506.1578, found [M+H]⁺ 506.1581.

### Example 38

N-(5-(3-(((3-fluorobenzyl)-12-nitren)sulfonyl)-4-methoxyphenyl)-4-methylthiazol-2-yl)hexanamide, with a structural formula as below:

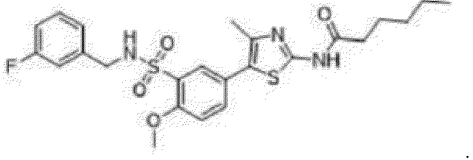

The starting material 1-(4-chlorophenyl)-2-acetone in Step 1 of Example 1 was replaced with 1-(4-methoxyphenyl)-2-acetone, the fragment tert-butyl (2-aminoethyl)carbamate in Step 2 was replaced with 3-fluorobenzylamine, and acetyl chloride in Step 4 was replaced with hexanoyl chloride, while other steps and procedures were identical to those in Example 1. A white solid, yield: 48%. ¹H NMR (400 MHz, DMSO-d₆) *δ* 12.10 (s, 1H), 8.03 (t,*J =* 6.5 Hz, 1H), 7.62 (d,J= 2.4 Hz, 1H), 7.54 (dd,J= 8.6, 2.4 Hz, 1H), 7.21-7.16 (m, 1H), 7.11 (d,J= 8.8 Hz, 1H), 7.00-6.91 (m, 3H), 4.12 (d,*J =* 6.5 Hz, 2H), 3.85 (s, 3H), 2.41 (t,*J =* 7.4 Hz, 2H), 2.28 (s, 3H), 1.62-1.56 (m, 2H), 1.38-1.16 (m, 4H), 0.85 (d,*J =* 7.1 Hz, 3H); ¹³C NMR (101 MHz, DMSO-d₆) *δ* 171.43, 163.11 (d,*J =* 244.4 Hz), 155.15, 155.02, 142.00, 140.54 (d,*J* = 7.1 Hz). 134.17, 129.89 (d,*J* = 9.1 Hz), 128.86 (d,J= 10.1 H1z), 123.81, 123.66 (d,*J* = 3.0 Hz), 122.38, 114.34, 114.12, 113.82 (d,J = 21.2 Hz), 113.15, 56.15, 45.77, 34.89, 30.77, 24.48, 21.89, 15.74, 14.02, 13.89; ESI-HRMS calcd for C₂₄H₂₈FN₃O₄S₂ m/z [M+H]⁺ 506.1578, found [M+H]⁺ 506.1584.

### Example 39

N-(5-(3-(((2-fluorobenzyl)-12-nitren)sulfonyl)-4-methoxyphenyl)-4-methylthiazol-2-yl)hexanamide, with a structural formula as below:

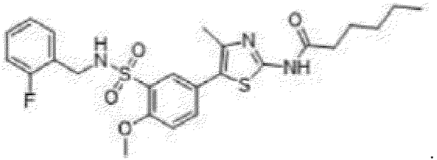

The starting material 1-(4-chlorophenyl)-2-acetone in Step 1 of Example 1 was replaced with 1-(4-methoxyphenyl)-2-acetone, and the fragment tert-butyl (2-aminoethyl)carbamate in Step 2 of Example 1 was replaced with 2-fluorobenzylamine, and acetyl chloride in Step 4 was replaced with hexanoyl chloride, while other steps and procedures were identical to those in Example 1; a white solid, yield: 42%. ¹H NMR (400 MHz, DMSO-d₆) *δ* 12.10 (s, 1H), 7.93 (t,J = 6.41 Hz, 1H), 7.63 (d,J= 2.4 Hz, 1H), 7.56-7.49 (m, 1H), 7.32 (td,*J* = 7.6, 1.8 Hz, 1H), 7.22-7.13 (m, 1H), 7.09-6.97 (m, 2H), 6.93-6.89 (m, 1H), 4.12 (dd,*J* = 28.1, 6.3 Hz, 2H), 3.82 (s, 3H), 2.41 (t,*J =* 7.4 Hz, 2H), 2.29 (s, 3H), 1.60 (p,*J =* 7.4 Hz, 2H), 1.36-1.21 (m, 4H), 0.90-0.81 (m, 3H); ¹³C NMR (101 MHz, DMSO-d₆) *δ* 171.41, 160.78 (d, *J =* 245.4 Hz), 155.09, 155.02, 141.94, 134.14, 130.37 *(d,J=* 3.0 Hz), 129.69 (d,J= 132.3 Hz), 129.33 (d,J= 8.1 Hz), 128.88, 124.29 (d,J= 15.2 Hz), 123.97 (d,*J =* 3.0 Hz), 123.72, 122.40, 114.79 (d,*J =* 21.2 Hz), 113.04, 56.06, 55.90 (d,*J =* 788.8 Hz), 34.88, 30.76, 24.47, 21.88, 15.71, 13.87; ESI-HRMS calcd for C₂₄H₂₈FN₃O₄S₂ m/z [M+H]⁺ 506.1578, found [M+H]⁺ 506.1575.

### Example 40

N-(4-(3-(((2,4-difluorobenzyl)-12-nitren)sulfonyl)-4-methoxyphenyl)-5-methylthiazol-2-yl)hexanamide, with a structural formula as below:

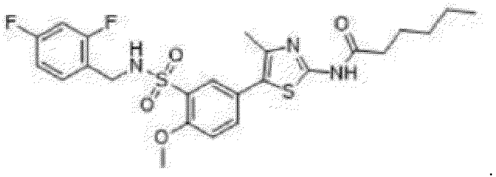

The starting material 1-(4-chlorophenyl)-2-acetone in Step 1 of Example 1 was replaced with 1-(4-methoxyphenyl)-2-acetone, the fragment tert-butyl (2-aminoethyl)carbamate in Step 2 was replaced with 2.4-difluorobenzylamine, and acetyl chloride in Step 4 was replaced with hexanoyl chloride, while other steps and procedures were identical to those in Example 1. A white solid, yield: 52%. ¹H NMR (400 MHz, DMSO-d₆) *δ* 12.09 (s, 1H), 7.92 (t,*J* = 6.4 Hz, 1H), 7.60 *(d,J =* 2.4 Hz, 1H), 7.54 (dd, *J* = 8.6, 2.4 Hz, 1H), 7.35 (td,J = 8.7, 8.3, 6.6 Hz, 1H), 7.07 (d,J = 8.7 Hz, 1H), 6.96-6.84 (m, 2H), 4.13 (d,*J* = 6.4 Hz, 2H), 3.83 (s, 3H), 2.42 (t,J= 7.4 Hz, 2H), 2.30 (s, 3H), 1.60 (p,*J =* 7.4 Hz, 2H), 1.32-1.25 (m, 4H), 0.87 (t,*J =* 6.9 Hz, 3H); ¹³C NMR (101 MHz, DMSO) *δ* 171.35, 162.73 (dd,*J* = 189.9, 12.1 Hz), 160.28 (dd,*J* = 192.9, 11.1 Hz), 154.98, 141.93, 134.04, 131.70 (dd,J= 15.2, 5.1 Hz), 128.79, 128.35, 123.73, 122.32, 120.67 (d,J = 4.0 Hz), 120.54 (m), 112.97, 111.09 (dd,*J* = 18.2, 2.0 Hz), 103.03 (t,*J* = 51.5 Hz), 56.04, 34.84, 30.72, 24.42, 21.82, 15.65, 13.82; ESI-HRMS calcd for C₂₄H₂₇F₂N₃O₄S₂ m/z [M+H]⁺ 524.1484, found [M+H]⁺ 524.1489.

### Example 41

N-(5-(3-(((3,4-difluorobenzyl)-12-nitren)sulfonyl)-4-methoxyphenyl)-4-methylthiazol-2-yl)hexanamide, with a structural formula as below:

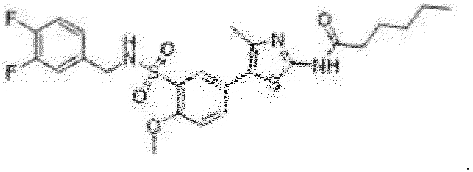

The starting material 1-(4-chlorophenyl)-2-acetone in Step 1 of Example 1 was replaced with 1-(4-methoxyphenyl)-2-acetone, the fragment tert-butyl (2-aminoethyl)carbamate in Step 2 was replaced with 3.4-difluorobenzylamine, and acetyl chloride in Step 4 was replaced with hexanoyl chloride, while other steps and procedures were identical to those in Example 1. A white solid, yield: 54%. ¹H NMR (400 MHz, DMSO-d₆) *δ* 12.09 (s, 1H), 8.00 (t,*J =* 6.5 Hz, 1H), 7.60 (d,*J =* 2.4 Hz, 1H), 7.55 (dd,*J* = 8.6, 2.4 Hz, 1H), 7.20-7.09 (m, 3H), 7.04-6.96 (m, 1H), 4.10 (d,*J =* 6.5 Hz, 2H), 3.86 (s, 3H), 2.41 (t,J= 7.4 Hz, 2H), 2.29 (s, 2H), 1.63-1.56 (m, 2H), 1.27 (dt,J= 10.3, 4.9, 4H), 0.87 (t,J= 6.9 Hz, 3H); ¹³C NMR (101 MHz, DMSO-d₆) *δ* 171.36, 155.02, 154.99, 141.96, 135.32 (dd,*J* = 4.0, 3.0 Hz), 134.05, 129.65, 128.82, 128.74, 124.50 (dd,J= 3.0, 3.0 Hz), 123.80, 122.29, 116.86 (d,J= 17.2 Hz), 116.62 (d,J= 17.2 Hz), 113.03, 56.12, 45.27, 34.85, 30.72, 24.42, 21.83, 15.65, 13.82; ESI-HRMS calcd for C₂₄H₂₇F₂N₃O₄S₂ m/z [M+H]⁺ 524.1484, found [M+H]⁺ 524.1480.

### Example 42

N-(5-(3-(((3-chloro-4-fluorobenzyl)-12-nitren)sulfonyl)-4-methoxyphenyl)-4-methylthiazol-2-yl)hexanamide, with a structural formula as below:

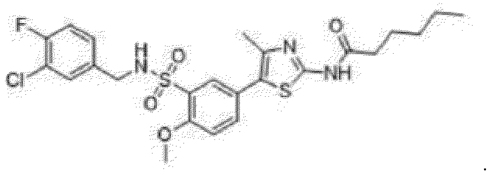

The starting material 1-(4-chlorophenyl)-2-acetone in Step 1 of Example 1 was replaced with 1-(4-methoxyphenyl)-2-acetone, the fragment tert-butyl (2-aminoethyl)carbamate in Step 2 was replaced with 3-chloro-4-fluorobenzylamine, and acetyl chloride in Step 4 was replaced with hexanoyl chloride, while other steps and procedures were identical to those in Example 1. A white solid, yield: 50%. ¹H NMR (400 MHz, DMSO-d₆) *δ* 12.08 (s, 1H), 8.00 (t,*J =* 6.6 Hz, 1H), 7.61-7.50 (m, 2H), 7.31 *(d,J=* 7.3 Hz, 1H), 7.15 *(d,J=* 7.3 Hz, 2H), 7.10 (d, *J=* 8.6 Hz, 1H), 4.11 *(d,J=* 6.5 Hz, 2H), 3.86 (s, 3H), 2.41 (t,*J =* 7.3 Hz, 2H), 2.29 (s, 3H), 1.63-1.56 (m, 2H), 1.32-1.25 (m, 4H), 0.86 (t,*J =* 6.8 Hz, 3H); ¹³C NMR (101 MHz, DMSO-d₆) *δ* 171.35, 157.31 (d,J= 247.4 Hz), 154.97, 154.94, 141.94, 135.29 *(d,J=* 3.0 Hz), 134.03, 129.66, 128.76, 128.70, 128.41 (d,*J =* 7.1 Hz), 123.84, 122.29, 118.86 (d,*J =* 17.2 Hz), 116.23 (d,*J* = 20.2 Hz), 112.96, 56.11, 45.14, 34.85, 30.72, 24.42, 21.82, 15.71, 13.82; ESI-HRMS calcd for C₂₄H₂₇CIFN₃O₄S₂ m/z [M+H]⁺ 540.1188, found [M+H]⁺ 540.1182.

### Example 43

N-(5-(3-(((4-fluoro-3-(trifluoromethyl)benzyl)-12-nitren)sulfonyl)-4-methoxyphenyl)-4-methylthiazol-2-yl)hexanamide, with a structural formula as below:

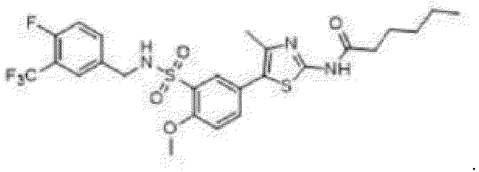

The starting material 1-(4-chlorophenyl)-2-acetone in Step 1 of Example 1 was replaced with 1-(4-methoxyphenyl)-2-acetone, the fragment tert-butyl (2-aminoethyl)carbamate in Step 2 was replaced with 3-chloro-4-(trifluoromethyl)benzylamine, and acetyl chloride in Step 4 was replaced with hexanoyl chloride, while other steps and procedures were identical to those in Example 1; a white solid, yield: 44%. ¹H NMR (400 MHz, DMSO-d₆) *δ* 12.08 (s, 1H), 8.05 (t,*J =* 6.5 Hz, 1H), 7.58 (d,*J =* 2.4 Hz, 1H), 7.56-7.50 (m, 3H), 7.32-7.24 (m, 1H), 7.08 (d,*J =* 8.7 Hz, 1H), 4.19 (d,*J =* 6.5 Hz, 2H), 3.83 (s, 3H), 2.41 (t,J= 7.4 Hz, 2H), 2.28 (s, 3H), 1.60 (p,*J =* 7.4 Hz, 2H), 1.36-1.18 (m, 4H), 0.87 (t,J= 6.9 Hz, 3H); ¹³C NMR (101 MHz, DMSO-d₆) *δ* 171.44, 156.56 *(d,J=* 194.9 Hz), 155.03, 154.90, 142.00, 134.65 (d,*J* = 54.5 Hz), 134.61 (d,*J =* 5.0 Hz), 130.20 (d,*J =* 49.5 Hz), 128.74, 128.67, 127.92, 126.60, 126.27 (m), 123.90, 122.29, 116.68 (d,J = 20.2 Hz), 113.00, 56.06, 45.15, 34.90, 30.79, 24.50, 21.91, 15.75, 13.90; ESI-HRMS calcd for C₂₅H₂₇F₄N₃O₄S₂ m/z [M+H]⁺ 574.1452, found [M+H]⁺ 574.1444.

### Example 44

N-(5-(4-methoxy-3-(((3,4,5-trifluorobenzyl)-12-nitren)sulfonyl)phenyl)-4-methylthiazol-2-yl)hexanamide, with a structural formula as below:

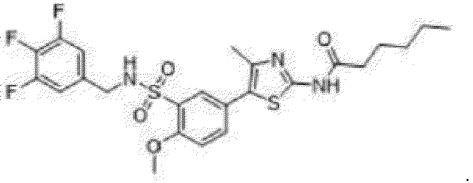

The starting material 1-(4-chlorophenyl)-2-acetone in Step 1 of Example 1 was replaced with 1-(4-methoxyphenyl)-2-acetone, the fragment tert-butyl (2-aminoethyl)carbamate in Step 2 was replaced with 3,4,5-trifluorobenzylamine, and acetyl chloride in Step 4 was replaced with hexanoyl chloride, while other steps and procedures were identical to those in Example 1. A white solid, yield: 31%. ¹H NMR (400 MHz, DMSO-d₆) *δ* 12.09 (s, 1H), 8.06 (t,*J =* 6.6 Hz, 1H), 7.60 (d,*J =* 2.3 Hz, 1H), 7.57 (dd,*J* = 8.5, 2.4 Hz, 1H), 7.14 (d,*J =* 8.7 Hz, 1H), 7.09 (dd,*J* = 8.9, 6.7 Hz, 2H), 4.12 (d,J= 6.6 Hz, 2H), 3.87 (s, 3H), 2.41 (t,*J*= 7.4 Hz, 2H), 2.28 (s, 3H), 1.60 (p,J= 7.2 Hz, 2H), 1.33-1.23 (m, 4H), 0.87 *(t,J=* 6.8 Hz, 3H); ¹³C NMR (101 MHz, DMSO-d₆) *δ* 171.37, 155.04, 154.99, 154.72, 141.98, 135.49, 134.15, 130.23, 128.87, 128.66, 127.85, 126.62, 123.87, 122.22, 112.96 (d,*J* = 72.7 Hz), 112.03 (m), 56.14 (d,*J* = 22.2 Hz), 47.94, 45.10, 45.00, 34.85, 30.72, 24.43, 21.83, 15.59, 13.82; ESI-HRMS calcd for C₂₄H₂₆F₃N₃O₄S₂ m/z [M+H]⁺ 542.1390, found [M+H]⁺ 542.1395.

### Example 45

N-(5-(3-(((4-fluorophenethyl)-12-nitren)sulfonyl)-4-methoxyphenyl)-4-methylthiazol-2-yl)hexanamide, with a structural formula as below:

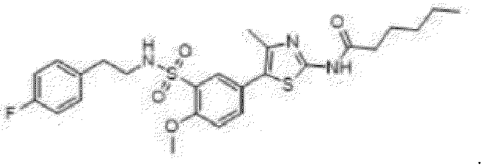

The starting material 1-(4-chlorophenyl)-2-acetone in Step 1 of Example 1 was replaced with 1-(4-methoxyphenyl)-2-acetone, the fragment tert-butyl (2-aminoethyl)carbamate in Step 2 was replaced with 4-fluorophenethylamine, and acetyl chloride in Step 4 was replaced with hexanoyl chloride, while other steps and procedures were identical to those in Example 1; a white solid, yield: 32%. ¹H NMR (400 MHz, DMSO-d₆) *δ* 12.12 (s, 1H), 7.70 (d,J = 2.4 Hz, 1H), 7.65 (dd,*J*= 8.6, 2.4 Hz, 1H), 7.43 (t,*J*= 5.8 Hz, 1H), 7.26 (d,*J =* 8.7 Hz, 1H), 7.17-7.13 (m, 2H), 7.06-7.01 (m, 2H), 3.89 (s, 3H), 3.06 (q,*J* = 6.8 Hz, 2H), 2.68 (t,*J =* 7.1 Hz, 2H), 2.41 (t,*J =* 7.4 Hz, 2H), 2.31 (s, 3H), 1.59 (p,*J =* 7.2 Hz, 2H), 1.27 (td,*J*= 8.5, 7.4, 5.0 Hz, 4H), 0.86 (t,*J*= 6.8 Hz, 3H); ¹³C NMR (101 MHz, DMSO-d₆) *δ* 171.54, 162.12 (d,*J* = 242.4 Hz), 155.38, 155.07, 142.11, 135.00 (d,*J =* 3.0 Hz), 134.22, 130.55, 130.47, 128.93 (d,*J*= 53.5 Hz), 124.05, 122.40, *115.10 (d,J* = 20.2 Hz), 113.65, 56.36, 44.20, 34.94, 34.49, 30.81, 24.53, 21.93, 15.87, 13.92; ESI-HRMS calcd for C₂₅H₃₀FN₃O₄S₂ m/z [M+H]⁺ 520.1735, found [M+H]⁺ 520.1735.

### Example 46

### Tert-butyl (2-(2-hexanamido-4-methylthiazol-5-yl)-2-methoxybenzamido)ethyl carbamate

### Step 1: Synthesis of 2-methoxy-5-(2-oxopropyl)benzoic acid

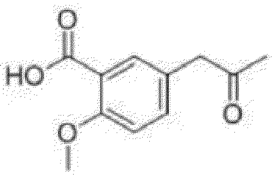

10 mmol methyl 5-formyl-2-methoxybenzoate and butylamine (2.0 eq.) as starting materials were dissolved in toluene and refluxed to allow a reaction to proceed for 3h; a residue was distilled under reduced pressure to dryness and dissolved in 10 mL of acetic acid; 1.14 mL of introethan (1.5 eq.) was slowly added to the mixture; and the mixture was heated to 100°C to allow a reaction to proceed for 3 h. Upon completion of the reaction, the reaction mixture was cooled to room temperature and slowly poured into 40 mL of an ice-water solution (with vigorous stirring throughout); a resulting mixture was extracted with ethyl acetate (2 × 40 mL) to collect organic phases; and the organic phases were washed successively with water (2 × 40 mL), a 10% sodium bicarbonate solution (2 × 30 mL) and saline, dried over anhydrous magnesium sulfate, and then purified by column chromatography (PE:EA = 2:1) to obtain methyl (Z)-2-methoxy-5-(2-nitropropen-1-yl)benzoate (yield: 80%). 100 mmol iron powder was added to a 22 mL reaction flask; under nitrogen atmosphere, 12 mL of an acetic acid solution containing dissolved methyl (Z)-2-methoxy-5-(2-nitropropen-1-yl)benzoate (8 mmol) was slowly added dropwise to the reaction flask; after a resulting mixture was refluxed to allow a reaction to proceed for 2 h, the reaction mixture was filtered to remove the iron powder; 30 mL of water was added to the obtained filtrate which was then extracted with an ethyl acetate solution (3 × 40 mL) to collect organic phases; the organic phases were washed successively with water (2 × 30 mL), a 10% sodium bicarbonate solution (2 × 30 mL) and saline (30 mL), dried over anhydrous magnesium sulfate, and then purified by column chromatography (PE:EA = 1:1) to obtain methyl 2-methoxy-5-(2-oxopropyl)benzoate (yield: 90%). 2 mmol methyl 2-methoxy-5-(2-oxopropyl)benzoate was metrologically weighed and subjected to acid hydrolysis to obtain 2-methoxy-5-(2-oxopropyl)benzoic acid as a white solid (yield: 95%). Here, a mixture of 1.0 mL of a hydrochloric acid solution (1 mol/L) and 4 mL of acetic acid was used as an acid hydrolysis solution. ¹H NMR (400 MHz, DMSO-d₆) *δ* 12.58 (s, 1H), 7.46 (d,*J =* 2.3 Hz, 1H), 7.30 (dd,*J* = 8.5, 2.4 Hz, 1H), 7.07 (d,*J =* 8.6 Hz, 1H), 3.80 (s, 3H), 3.75 (s, 2H), 2.13 (s, 3H) Step 2: Synthesis of tert-butyl (2-(2-hexanamido-4-methylthiazol-5-yl)-2-methoxybenzamido)ethyl carbamate, with a structural formula as below:

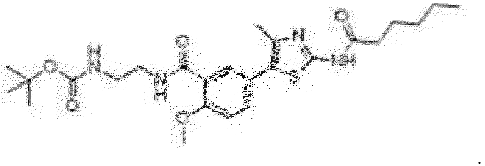

2-methoxy-5-(2-oxopropyl)benzoic acid (1 mmol) and CDI (1 mmol) were dissolved in 10 mL of DCM solution to allow a pre-reaction to proceed for 1 h, followed by adding 1.5 mmol tert-butyl (2-aminoethyl) carbamate to the reaction mixture to allow a reaction to proceed at room temperature. Upon completion of the reaction, tert-butyl (2-(2-methoxy-5-(2-oxopropyl)benzamido)ethyl)carbamate was obtained; and then tert-butyl (2-(2-hexanamido-4-methylthiazol-5-yl)-2-methoxybenzamido)ethyl carbamate as a white solid (yield: 40%) was obtained following Steps 3 and 4 in Example 1. ¹H NMR (400 MHz, DMSO-d₆) *δ* 12.12 (s, 1H), 7.73 (d,J= 2.3 Hz, 1H), 7.68 (dd,J= 8.6, 2.4 Hz, 1H), 7.32 (d,J= 8.7 Hz, 1H), 7.16-7.04 (m, 1H), 3.96 (s, 3H), 3.45 (t,*J =* 4.7 Hz, 4H), 2.96 (q,*J* = 6.3 Hz, 2H), 2.42 (t,*J =* 7.4 Hz, 2H), 2.32 (s, 3H), 2.28-2.19 (m, 6H), 1.79-1.73 (m, 2H), 1.37-1.17 (m, 4H), 0.90-0.85 (m, 3H); ¹³C NMR (101 MHz, DMSO) *δ* 155.35, 154.96, 136.08, 134.65, 130.56, 129.14, 126.96, 126.53, 113.86, 112.90, 63.17, 56.35, 55.51, 51.35, 47.92, 36.94, 30.70, 24.40, 21.80, 15.85, 13.82. ESI-HRMS calcd for C₂₃H₃₄N₄O₅S₂ m/z [M+H]⁺ 511.2043, found [M+H]⁺ 511.2044.

### Example 47

N-(4-(3-(((2,4-difluorobenzyl)-12-nitren)sulfonyl)-4-methoxyphenyl)-5-methylthiazol-2-yl)hexanamide, with a structural formula as below:

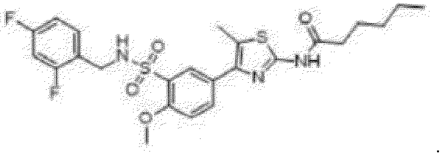

This example was similar to the method in Example 1, except that the starting material 1-(4-chlorophenyl)-2-acetone in Step 1 of Example 1 was replaced with 4'-methoxypropiophenone, the fragment tert-butyl (2-aminoethyl)carbamate in Step 2 was replaced with 2.4-difluorobenzylamine, and acetyl chloride in Step 4 was replaced with hexanoyl chloride, while other steps and procedures were identical to those in Example 1. a white solid, yield: 51%. ¹H NMR (300 MHz, DMSO-d₆) *δ* 12.08 (s, 1H), 8.01 *(d,J =* 2.3 Hz, 1H), 7.84 (d,*J* = 6.4 Hz, 1H), 7.75 (dd,*J* = 8.7, 2.3 Hz, 1H), 7.39-7.31 (m, 1H), 7.09 (d,*J =* 8.7 Hz, 1H), 6.97-6.89 (m, 2H), 4.09 (d,*J =* 6.3 Hz, 2H), 3.84 (s, 3H), 2.45 (s, 3H), 2.40 (t,J = 7.4 Hz, 2H), 1.64-1.56 (m, 2H), 1.26 (td,J= 6.4, 6.0, 3.0 Hz, 4H), 0.89-0.84 (m, 3H); ¹³C NMR (101 MHz, DMSO) *δ* 171.33, 162.77 (d,*J* = 204.0 Hz), 160.17 (d,*J* = 177.8 Hz), 154.88, 154.01, 142.26, 132.99, 131.58 (d,*J* = 6.1 Hz), 128.78, 127.96, 126.88, 120.88 (dd,*J* = 15.2, 3.0 Hz), 120.26, 112.27, 111.08 (dd,*J* = 18.2, 3.0 Hz), 103.12 (t,*J* = 52.5 Hz), 55.98, 34.81, 30.74, 24.43, 21.82, 13.82. 11.77; ESI-HRMS calcd for C₂₄H₂₇F₂N₃O₄S₂ *m*/*z* [M+H]⁺ 524.1484, found [M+H]⁺ 524.1491. PI4KIIIβ inhibition assays were conducted for the inhibitors of the present invention using ADP-Glo Luminescent Kinase Assays, and reagents used in the kinase reaction were as follows: HEPES (50 mM) pH 7.5 with NaCl (100 mM), EGTA (1.0mM), MgCl₂ (3.0 mM), DTT (2.0 mM) and CHAPS (0.03%). During the reaction, 50 µM PIP2 and 25 µM ATP were added to 10 mL of each test compound at varying concentrations (0.05 nM to 1.0 µM). A reaction mixture was incubated at room temperature for 1 h, and then, 10 µL of an ADP-Glo reagent was added to terminate an enzymatic reaction. Data were collected using Envision software, and the IC₅₀ values of the compounds were analyzed and fitted using Graphpad Prism 5.

**Table 1: PI4KIIIβ inhibitory activity (IC₅₀, nM) of compounds of examples**

| Example | Example 1 | Example 2 | Example 3 | Example 4 | Example 5 | Example 6 | Example 7 | Example 8 | Example 9 | Example 10 |
|---|---|---|---|---|---|---|---|---|---|---|
| PI4KIIIβ | 19 | 44 | 61 | 61 | 16 | 38 | 15 | 32 | 10 | 5.7 |

| Example | Example 11 | Example 12 | Example 13 | Example 14 | Example 15 | Example 16 | Example 17 | Example 18 | Example 19 | Example 20 |
|---|---|---|---|---|---|---|---|---|---|---|
| PI4KIIIβ | 4.6 | 63 | 10 | 63 | 13 | 32 | 77 | 32 | 10 | 5.7 |

| Example | Example 21 | Example 22 | Example 23 | Example 24 | Example 25 | Example 26 | Example 27 | Example 28 | Example 29 | Example 30 |
|---|---|---|---|---|---|---|---|---|---|---|
| PI4KIIIβ | 4.6 | 89 | 8.2 | 68 | 8.4 | 77 | 4.9 | >100 | 37 | >100 |

| Example | Example 31 | Example 32 | Example 33 | Example 34 | Example 35 | Example 36 | Example 37 | Example 38 | Example 39 | Example 40 |
|---|---|---|---|---|---|---|---|---|---|---|
| PI4KIIIβ | 58 | 17 | 4.9 | >100 | 56 | 180 | 51 | 46 | 24 | 19 |

| Example | Example 41 | Example 42 | Example 43 | Example 44 | Example 45 | Example 46 | Example 47 | PIK-93 | | |
|---|---|---|---|---|---|---|---|---|---|---|
| PI4KIIIβ | 66 | 193 | >500 | 61 | 33 | 7.4 | >500 | 17 | | |

As shown in Table 1, the compounds of the present invention exhibit a nanomolar-level inhibitory activity against PI4KIIIβ, and some of the compounds are significantly superior to the positive control PIK-93. In particular, Examples 10, 11, 12, 14, 20, 21, 24, 27 and 33 exhibit significantly greater inhibitory activity against PI4KIIIβ as compared to the positive control PIK-93. This indicates that the compounds in the examples of the present invention are efficient PI4KIIIβ inhibitors.

Anti-proliferative activity assays in tumor cell lines were conducted for the inhibitors of the present invention. The inhibitory activity of the compounds against cell proliferation was evaluated using the CCK 8 assay, and initial single-concentration screening and multi-concentration assays were conducted to determine the half-maximal inhibitory concentration (IC₅₀) values. Detection principle: the cytotoxicity (CCK 8 assay) detection principle was as follows: a CCK 8 reagent contained WST-8, which was reduced by dehydrogenases in cell mitochondria under the action of an electron carrier 1-methoxy-5-methylphenazine dimethyl sulfate (1-Methoxy PMS) to produce a highly water-soluble yellow formazan product. The amount of the produced formazan product was directly proportional to the number of live cells.

Experimental methods were as follows.
(1) Cell seeding: a single-cell suspension was prepared using a culture medium containing 10% fetal bovine serum; 90 µL of adherent cells (5 × 10⁴/mL) and suspension cells (9 × 10⁴/mL) was seeded into each well of a 96-well plate; and the cells were pre-incubated for 24 h at 37°C in the presence of 5% CO₂.
(2) Addition of test sample solution: 10 µL of a sample solution was added to each well; for initial activity screening, one concentrate was set per sample, with three replicates; for IC₅₀ determination, eight concentrations (including the concentration of 0) were used, with three replicates set per concentration; and the incubation was conducted for 48 h in an incubator. In this experiment, a blank group (Blank), a control group (Control) and a drug group (Drug) were provided.
(3) Color development: For the adherent cells, the old culture medium and the drug solution were aspirated (for suspension cells, they were directly added to 10 µL of undiluted CCK 8 solution); 100 µL of the CCK 8 solution diluted 10-fold was added to each well; and the incubation was continued for 14 h at 37°C in the presence of 5% CO₂ (with the procedures monitored in real time).
(4) Detection: The absorbance at 450 nm was measured using a microplate reader, and raw data results were recorded.
(5) The raw data were standardized using Excel software; the cell proliferation inhibition rate in each well during initial screening was calculated using the OD value (via the formula: inhibition rate = (OD_{control} - OD_{Drug})/(OD_{control} - OD_{Blank}) × 100%), and the statistical analysis of the inhibition rates was performed. IC₅₀ was calculated using GraphPad Prism 8.

**Table 2: Anti-proliferative activity (IC₅₀, µM) of compounds of examples against tumor cell lines**

| Example | MCF-7 | MDA-MB-231 | A549 | H446 | DU145 | PC3 | HT29 | HCT116 |
|---|---|---|---|---|---|---|---|---|
| Example 1 | 2.36 | 2.05 | 2.91 | 0.44 | 1.97 | 1.24 | 2.04 | 1.54 |
| Example 2 | 2.83 | 2.87 | 3.3 | 0.79 | 3.53 | 2.98 | 2.64 | 2.31 |
| Example 3 | >4 | >4 | >4 | 2.09 | >4 | >4 | >4 | >4 |
| Example 4 | >4 | >4 | >4 | 0.80 | >4 | >4 | >4 | >4 |
| Example 5 | 2.11 | 3.94 | 3.87 | 0.70 | >4 | 2.4 | 2.98 | 2.38 |
| Example 6 | >4 | >4 | >4 | 3.32 | >4 | >4 | >4 | 3.89 |
| Example 7 | >4 | >4 | >4 | >4 | >4 | >4 | >4 | >4 |
| Example 8 | 1.16 | 1.04 | 1.87 | 0.21 | 1.51 | 0.84 | 0.80 | 0.38 |
| Example 9 | 0.42 | 0.62 | 0.54 | 0.094 | 0.56 | 0.42 | 0.16 | 0.29 |
| Example 10 | 0.31 | 0.49 | 0.48 | 0.067 | 0.43 | 0.28 | 0.12 | 0.25 |
| Example 11 | 0.097 | 0.15 | 0.16 | 0.038 | 0.082 | 0.074 | 0.049 | 0.74 |
| Example 12 | 0.14 | 0.18 | 0.24 | 0.05 | 0.12 | 0.13 | 0.078 | 0.14 |
| Example 13 | 0.68 | 0.54 | 0.95 | 0.14 | 0.87 | 0.63 | 0.45 | 0.60 |
| Example 14 | 0.50 | 0.47 | 0.63 | 0.095 | 0.58 | 0.45 | 0.28 | 0.35 |
| Example 15 | 0.34 | 0.44 | 0.74 | 0.062 | 0.31 | 0.24 | 0.05 | 0.29 |
| Example 16 | >2 | >2 | >2 | 0.71 | >2 | >2 | 1.45 | >2 |
| Example 17 | >2 | >2 | >2 | 1.10 | >2 | >2 | >2 | >2 |
| Example 18 | 0.36 | 0.46 | 0.81 | 0.058 | 0.32 | 0.24 | 0.064 | 0.38 |
| Example 19 | 0.75 | 0.93 | 1.56 | 0.17 | 0.74 | 0.43 | 0.062 | 0.64 |
| Example 20 | 0.55 | 0.68 | 1.59 | 0.11 | 0.53 | 0.38 | 0.10 | 0.42 |
| Example 21 | 0.77 | 1.15 | 1.98 | 0.16 | 1.35 | 0.70 | 0.24 | 0.99 |
| Example 22 | 0.86 | 0.77 | 0.92 | 0.071 | 0.48 | 0.37 | 0.094 | 0.53 |
| Example 23 | 1.10 | 0.91 | 0.86 | 0.042 | 0.40 | 0.31 | 0.076 | 0.18 |
| Example 24 | 1.17 | 1.92 | 1.38 | 0.07 | 0.94 | 0.64 | 0.096 | 0.36 |
| Example 25 | 1.04 | 1.07 | 1.21 | 0.062 | 0.64 | 0.45 | 0.078 | 0.28 |
| Example 26 | >2 | >2 | >2 | >2 | >2 | >2 | >2 | >2 |
| Example 27 | 0.28 | 0.39 | 0.42 | 0.086 | 0.29 | 0.31 | 0.096 | 0.23 |
| Example 28 | >2 | >2 | >2 | >2 | >2 | >2 | >2 | >2 |
| Example 29 | >4 | >4 | >4 | 0.45 | >4 | 0.92 | 0.70 | >4 |
| Example 30 | 0.89 | 1.47 | 1.86 | 0.32 | 1.28 | 0.67 | 0.52 | 0.80 |
| Example 31 | >4 | >4 | >4 | >4 | >4 | >4 | >4 | >4 |
| Example 32 | >4 | >4 | >4 | >4 | >4 | >4 | >4 | >4 |
| Example 33 | >4 | >4 | >4 | >4 | >4 | >4 | >4 | >4 |
| Example 34 | 0.82 | 1.57 | >2 | 0.34 | 1.73 | 0.76 | 0.52 | 1.46 |
| Example 35 | 0.46 | 0.87 | 2.76 | 0.20 | 0.32 | 0.21 | 0.14 | 0.52 |
| Example 36 | 1.05 | 2.41 | >4 | 0.43 | 0.57 | 0.84 | 0.38 | 0.94 |
| Example 37 | 0.20 | 0.34 | 0.59 | 0.12 | 0.37 | 0.19 | 0.18 | 0.20 |
| Example 38 | 0.21 | 0.74 | 1.24 | 0.35 | 0.68 | 0.43 | 0.42 | 0.38 |
| Example 39 | 0.20 | 0.68 | 1.07 | 0.24 | 0.53 | 0.22 | 0.31 | 0.27 |
| Example 40 | 0.13 | 0.18 | 0.24 | 0.098 | 0.19 | 0.16 | 0.14 | 0.15 |
| Example 41 | 0.20 | 0.28 | 0.34 | 0.22 | 0.30 | 0.21 | 0.25 | 0.19 |
| Example 42 | 0.19 | 0.26 | 0.30 | 0.18 | 0.24 | 0.19 | 0.21 | 0.24 |
| Example 43 | 0.32 | 0.53 | 0.79 | 0.30 | 0.60 | 0.24 | 0.40 | 0.29 |
| Example 44 | 0.21 | 0.35 | 0.40 | 0.38 | 0.42 | 0.18 | 0.17 | 0.19 |
| Example 45 | 0.42 | 1.02 | 1.84 | 0.16 | 0.87 | 0.48 | 0.46 | 0.75 |
| Example 46 | >2 | >2 | >2 | >2 | >2 | >2 | >2 | >2 |
| Example 47 | >4 | >4 | >4 | >4 | >4 | >4 | >4 | >4 |
| PIK-93 | 3.30 | 3.12 | >4 | 0.68 | >4 | 3.53 | 1.94 | 3.73 |

As can be seen from Table 2, the compounds of the present invention exhibit a micromolar-level anti-proliferative activity against various tumor cell lines, and in particular, most of the compounds of the examples show high sensitivity towards the human small cell lung cancer cell line H446 and the human colon cancer cell line HT29. Here, 12 compounds including that of Example 9 exhibit a nanomolar-level inhibitory activity against various cell lines in Table 3, significantly surpassing that of the positive control PIK-93. Here, the compound of Example 11 exhibits a nanomolar-level inhibitory activity against 6 out of 8 cell lines in Table 3, with the inhibitory activities of 38 nM and 49 nM against the cell lines H446 and HT29, respectively, representing significant enhancement over the positive control PIK-93. As can be seen from this, the compounds in the examples of the present invention can be potentially applied to the clinical treatment of the tumors described above.

Pharmacokinetic experiments on the PI4KIIIβ inhibitors of the present invention were conducted as follows: a test sample was metrologically weighed and added to a sterile vial; 250 µL of DMSO was added, and then,10 µL of methanesulfonic acid was added; after dissolution, 4.78 mL of 5% glucose injection was added; a resulting mixture was ultrasonically treated and shaken to mix thoroughly to prepare a 2 mg/mL test sample solution, which was used as a formulation for intragastric administration. In addition, 0.5 ml of 2 mg/mL test sample solution was taken; 4.5 mL of 5% glucose injection was added; a resulting mixture was shaken to mix thoroughly to prepare a 0.2 mg/mL test sample solution, which was used as a formulation for intravenous injection administration.

20 SD rats were divided into four groups. Due to the structural instability of the compounds of Examples 11 and 27 under acidic conditions, the compounds of Examples 11 and 27 were administered only via tail vein injection (5mg/kg), and the compound of Example 40 was administered via tail vein injection (5 mg/kg) and intragastrically (50 mg/kg), respectively. 0.25 mL of blood samples was collected from the retroorbital venous plexus 2 min, 5 min, 15 min, 30 min, 1 h, 2 h, 4 h, 6 h, 8 h and 12 h after administration to the intravenous injection group, and 5 min, 15 min, 30 min, 1 h, 2 h, 4 h, 6 h, 8 h, 12 h and 24 h after administration to the intragastric group. The concentrations of the compounds of Examples 11, 27 and 40 in SD rat plasma samples were determined using LC-MS/MS, and pharmacokinetic parameters were calculated using WinNolin software, with the results shown in Table 3.

As shown in Table 3, the compounds of Examples 11, 27 and 40 of the present invention are well metabolized in rats, exhibiting good absorption and exposure, and the compound of Example 40 exhibits high bioavailability.

**Table 3: Records of pharmacokinetic parameters**

| Example | Route of administration | Cₘₐₓ (µg/mL) | CL (L/h/Kg) | V_{SS} (L/Kg) | V_{Z} (L/Kg) | T_{1/2} (h) | MRT_{INF} (h) | AUC₀₋ₜ (h·ng/mL) | AUC_{0-∞} (h·ng/mL) | *F* (%) |
|---|---|---|---|---|---|---|---|---|---|---|
| 11 | iv | 3.75 | 0.38 | 0.51 | 0.79 | 1.49 | 1.41 | 2577.36 | 2700.26 | |
| 27 | iv | 1.92 | 0.8 | 1.01 | 1.24 | 1.22 | 1.51 | 2201.00 | 2261.04 | |
| 40 | iv | 2.98 | 0.44 | 0.59 | 0.94 | 1.50 | 1.35 | 2249.14 | 2418.41 | 49.9 |
| | po | 2.96 | | | 17 | 2.60 | 4.08 | 10579.92 | 12069.28 | |

Anti-tumor efficacy and toxicity assessment experiments of the PI4KIIIβ inhibitors of the present invention were conducted.
Drugs were derived from Examples 11, 27 and 40. The cell line used was human small cell lung cancer cell line H446, which was cultured in an RPMI-1640 culture medium containing 10% fetal bovine serum. Test animals were SPF-grade BALB/c nude male mice, with 5 mice per group; and the drug dosages were set out as in Table 4.

**Table 4: Drug dosage regimens**

| Group | Test drug | Dose (mg/kg) | Volume administered (mL/20 body weight) | Route of administration | Observation period (days) |
|---|---|---|---|---|---|
| Model group 1 | Normal saline | | 0.2 | Injection | 25 |
| Example 11 low-dose group | Example 11 | 20 | 0.2 | Injection | 25 |
| Example 11 high-dose group | Example 11 | 30 | 0.2 | Injection | 25 |
| Example 27 | Example 27 | 30 | 0.2 | Injection | 25 |
| Model group 2 | Normal saline | - | 0.2 | Intragastric | 25 |
| Example 40 low-dose group | Example 40 | 75 | 0.2 | Intragastric | 25 |
| Example 40 high-dose group | Example 40 | 150 | 0.2 | Intragastric | 25 |

### Drug preparation method:

Example 11 (20 mg/kg): 4 mg of test compound powder was dissolved in 2 mL of normal saline to prepare a 2 mg/mL solution, which was administered via oral administration and injection at a volume of 0.2 mL per 20 g of body weight.

Example 11 (30 mg/kg): 6 mg of test compound powder was dissolved in 2 mL of normal saline to prepare a 3 mg/mL solution, which was administered via oral administration and injection at a volume of 0.2 mL per 20 g of body weight.

Example 27 (30 mg/kg): 6 mg of test compound powder was dissolved in 2 mL of normal saline to prepare a 3mg/mL solution, which was administered via oral administration and injection at a volume of 0.2 mL per 20 g of body weight.

Example 40 (75 mg/kg): 15 mg of test compound powder was dissolved in 2 mL of normal saline to prepare a 7.5 mg/mL solution, which was administered via oral gavage at a volume of 0.2 mL per 20 g of body weight.

Example 40 (150 mg/kg): 30 mg of test compound powder was dissolved in 2 mL of normal saline to prepare a 15 mg/mL solution, which was administered via oral gavage at a volume of 0.2 mL per 20 g of body weight.

Experimental method: A human lung cancer xenograft model in nude mice was established by subcutaneous inoculation of the human small cell lung cancer cell line H446 into the axilla of nude mice. H446 cells in the logarithmic growth phase were subcutaneously inoculated into the right axillae of 50 nude mice under sterile conditions, at a dose of 5 × 10⁶ cells/mouse. The diameter of each transplanted tumor was measured using a Vernier caliper; after the tumors grew to approximately 80 mm², 42 tumor-bearing nude mice with good growth status and uniform tumor size were selected and randomized into 7 groups (with 6 mice per group), including a model group 1, an Example 11 low-dose group (20 mg/kg), an Example 11 high-dose group (30 mg/kg), an Example 27 group (30 mg/kg), a model group 2, an Example 40 low-dose group (75 mg/kg), and an Example 40 high-dose group (150 mg/kg). The Example 11 low- and high-dose groups and the Example 27 group were administered with the test drug by injection, while the Example 40 low- and high-dose groups were administered intragastrically, with administration every two days. The model groups 1 and 2 were administered with a vehicle as a control at equal volume. The anti-tumor effects of the test compounds were dynamically observed by measuring the tumor diameter. The tumor diameter was measured every other day, and the body weight of each nude mouse was weighed at the same time. On Day 27, the mice were sacrificed, and tumor masses were surgically excised, fixed in 10% formaldehyde and stored in liquid nitrogen for later use.

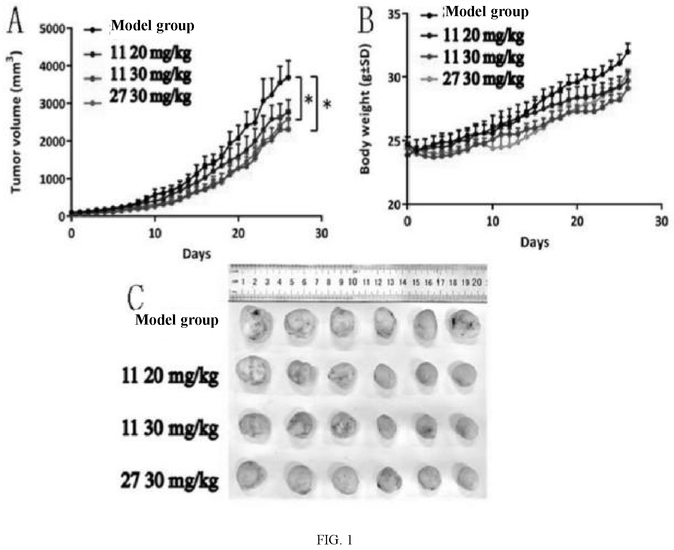

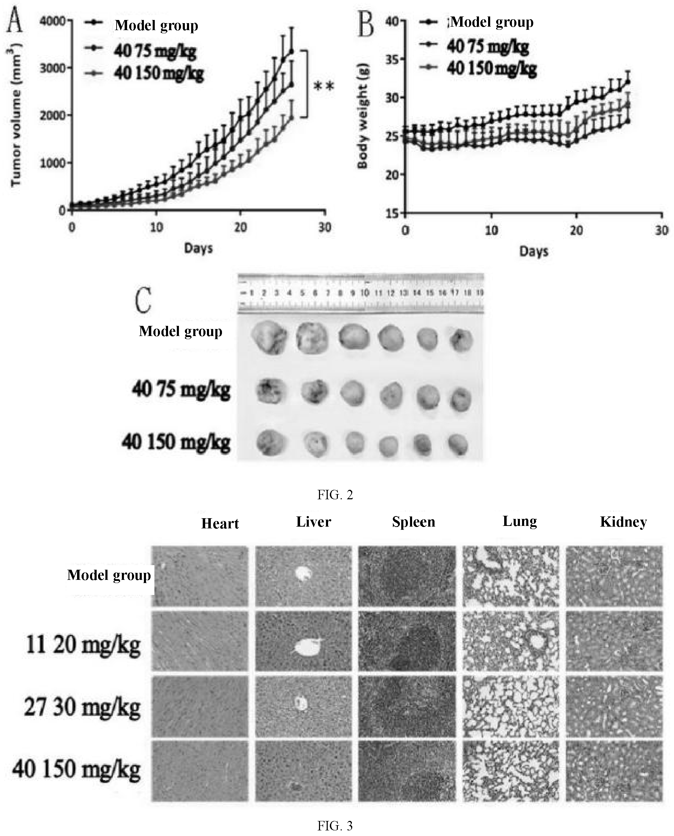

The experimental results show that the test drug of Example 11 exhibits an effective anti-tumor activity at 20 mg/kg, with a tumor growth inhibition (TGI) value of 25.7% and a relative tumor proliferation rate (T/C) of 72.3% compared to the model group. When the dose is increased to 30mg/kg, the tumor growth inhibition effect becomes better (TGI = 37.1%, T/C = 60.2%), which is far more than that of Example 27 (TGI = 30.2% and T/C = 66.8%). Furthermore, the compound of Example 40 exhibits more significant anti-tumor efficacy at an oral dose of 150 mg/kg. The TGI value is 42.2%, and the T/C value is 52.1%. FIG. 1 to FIG. 3 show the results of in vivo anti-tumor efficacy and toxicity assessment of the PI4KIIIβ inhibitors, respectively. FIG. 1 and FIG. 2 include the growth curves of tumor volume and body weight in mice as well as the representative images of tumor tissues from the mice, and FIG. 3 shows the representative images captured from the histopathological sections of main tissues harvested from tumor-bearing mice, with the histopathological sections stained with hematoxylin and eosin (H&E). In conjunction with the drawings described above, it can be seen that the test drugs prepared from Examples 11 and 40 show a significant inhibitory effect against the growth of human lung cancer H446 xenografts in nude mice.

The phenylthiazolamine-based PI4KIIIβ inhibitor of the present invention is highly effective for inhibiting PI4KIIIβ and the proliferation of various solid and hematological tumor cells, is highly sensitive to small cell lung cancer and colorectal cancer and has anti-tumor efficacy, showing potential for clinical tumor treatment

## Claims

1. A phenylthiazolamine-based PI4KIIIβ inhibitor, wherein the inhibitor is a substituted phenylthiazolamine-based compound of Formula V, or a stereoisomer, hydrate or pharmaceutically acceptable salt thereof: 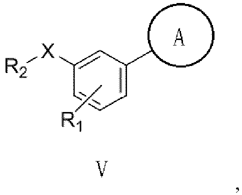 wherein A is 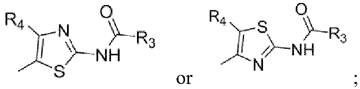 R₁ is a substituent on a benzene ring selected from the group consisting of hydrogen, fluorine, chlorine, bromine, iodine, hydroxy, amino, cyano, C1-C6 alkyl, C1-C6 haloalkyl, C1-C6 hydroxyalkyl, C1-C6 alkoxy, C1-C6 haloalkoxy, C1-C6 hydroxyalkoxy or C1-C6 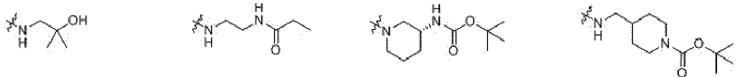 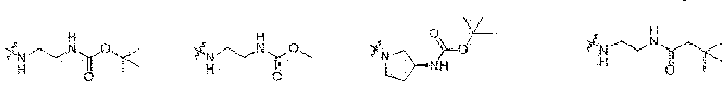 alkoxy C1-C6 alkyl; R₂ is 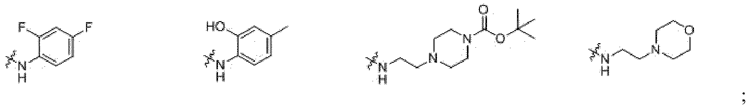 R₃ or R₄ is C1-C6 alkyl, C1-C6 alkyl substituted with one or more substituents, C1-C6 alkoxy, C1-C6 alkoxy substituted with one or more substituents, C1-C6 alkylacyl, C1-C6 alkylsulfonyl, C3-C6 heterocyclyl, or C3-C6 heterocyclyl substituted with one or more substituents; and
X is sulfonyl or carbonyl.

2. The inhibitor according to claim 1, wherein the inhibitor is a compound of Formula I or II: 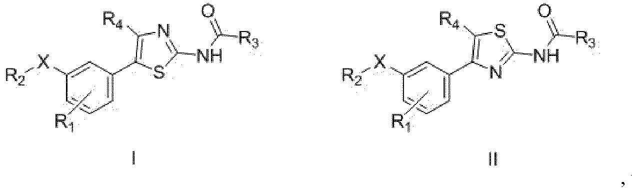 wherein R₁, R₂, R₄ and X are defined as 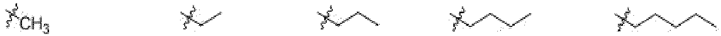 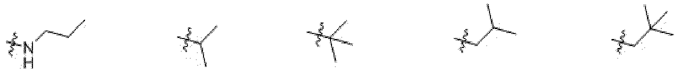 in claim 1; and R₃ is

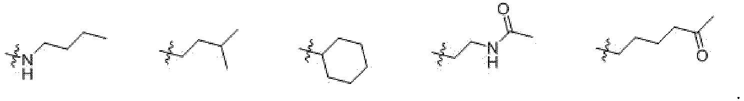

3. The inhibitor according to claim 1, wherein in the inhibitor of Formula V, hydrogen attached to carbon is replaced by deuterium, which is an isotope of the hydrogen.

4. The inhibitor according to any one of claims 1 to 3, wherein the inhibitor is one of:
(1) tert-butyl (2-(5-(2-acetamido-4-methylthiazol-5-yl)-2-chlorophenylsulfonamido)ethyl)carbamate;
(2) N-(5-(4-chloro-3-(N-(2-hydroxy-2-methylpropyl)aminosulfonyl)phenyl)-4-methylthiazol-2-yl)acetamide;
(3) N-(2-(2-acetamido-4-methylthiazol-5-yl)-2-chlorophenylsulfonamido)ethylpropionamide;
(4) methyl (2-(2-acetamido-4-methylthiazol-5-yl)-2-chlorophenylsulfonamido)ethyl carbamate;
(5) tert-butyl (2-((5-(2-acetamido-4-methylthiazol-5-yl)-2-methoxyphenyl)sulfonamido)ethyl)carbamate;
(6) N-(5-(4-chloro-3-(N-(3-hydroxy-4-methylphenyl)aminosulfonyl)phenyl)-4-methylthiazol-2-yl)acetamide;
(7) N-(5-(3-(N-(2.4-difluorophenyl)aminosulfonyl)-4-methoxyphenyl)-4-methylthiazol-2-yl)acetamide;
(8) tert-butyl (5-(2-propionamido-4-methylthiazol-5-yl)-2-methoxyphenylsulfonamido)ethyl carbamate;
(9) tert-butyl (5-(2-butanamido-4-methylthiazol-5-yl)-2-methoxyphenylsulfonamido)ethyl carbamate;
(10) tert-butyl (5-(2-pentanamido-4-methylthiazol-5-yl)-2-methoxyphenylsulfonamido)ethyl carbamate;
(11) tert-butyl (5-(2-hexanamido-4-methylthiazol-5-yl)-2-methoxyphenylsulfonamido)ethyl carbamate;
(12) tert-butyl (5-(2-heptanamido-4-methylthiazol-5-yl)-2-methoxyphenylsulfonamido)ethyl carbamate;
(13) tert-butyl (2-(2-methoxy-5-(4-methyl-2-(3-propylureido)thiazol-5-yl)phenyl)sulfonamido)ethyl carbamate;
(14) tert-butyl (2-(5-(2-(3-butylureido)-4-methylthiazol-5-yl)-2-methoxyphenylsulfonamido)ethyl)carbamate;
(15) tert-butyl (2-(2-methoxy-5-(4-methyl-2-(3-methylbutanamido)thiazol-5-yl)phenyl)sulfonamido)ethyl carbamate;
(16) tert-butyl (5-(2-isobutanamido-4-methylthiazol-5-yl)-2-methoxyphenylsulfonamido)ethyl carbamate;
(17) tert-butyl (2-(2-methoxy-5-(4-methyl-2-(2,2-dimethylpropanamido)thiazol-5-yl)phenyl)sulfonamido)ethyl carbamate;
(18) tert-butyl (5-(2-(3,3-dimethylbutanamido)-4-methylthiazol-5-yl)-2-methoxyphenylsulfonamido)ethyl carbamate;
(19) tert-butyl (2-(2-methoxy-5-(4-methyl-2-(4-methylpentanamido)thiazol-5-yl)phenyl)sulfonamido)ethyl carbamate;
(20) tert-butyl (2-(2-methoxy-5-(4-methyl-2-(5-methylhexanamido)thiazol-5-yl)phenyl)sulfonamido)ethyl carbamate;
(21) tert-butyl (2-(5-(2-(cyclohexanecarboxamido)-4-methylthiazol-5-yl)-2-methoxyphenyl)sulfonamido)ethyl carbamate;
(22) tert-butyl (2-(2-chloro-5-(4-methyl-2-(3-methylbutanamido)thiazol-5-yl)phenyl)sulfonamido)ethyl carbamate;
(23) tert-butyl (2-chloro-5-(2-(3,3-dimethylbutanamido)-4-methylthiazol-5-yl)phenylsulfonamido)ethyl carbamate;
(24) tert-butyl (2-chloro-5-(4-methyl-2-pentanamido-5-yl)phenylsulfonamido)ethyl carbamate;
(25) tert-butyl (2-chloro-5-(2-hexanamido-4-methylthiazol-5-yl)phenylsulfonamido)ethyl carbamate;
(26) tert-butyl (2-(5-(2-(3-acetamidopropionamido)-4-methylthiazol-5-yl)-2-methoxyphenyl)sulfonamido)ethyl carbamate;
(27) tert-butyl (2-(2-methoxy-5-(4-methyl-2-(6-oxoheptanamido)thiazol-5-yl)phenyl)sulfonamido)ethyl carbamate;
(28) tert-butyl (2-(2-methoxy-5-(4-methyl-2-(6-oxohexanamido)thiazol-5-yl)phenyl)sulfonamido)ethyl carbamate;
(29) tert-butyl (R)-(1-((5-(2-hexanamido-4-methylthiazol-5-yl)-2-methoxyphenyl)sulfonyl)piperidin-3-yl)carbamate;
(30) tert-butyl (S)-(1-((5-(2-hexanamido-4-methylthiazol-5-yl)-2-methoxyphenyl)sulfonyl)pyrrolidin-3-yl)carbamate;
(31) tert-butyl 4-((2-hexanamido-4-methylthiazol-5-yl)-2-methoxyphenylsulfonamido)piperidin-1-carboxylate;
(32) N-(5-(3-(2-(3,3-dimethylbutanamido)ethylaminosulfonyl)-4-methoxyphenyl)-4-methylthiazol-2-yl)hexanamide;
(33) N-(5-(3-(2-hydroxy-2-methylpropyl)aminosulfonyl)-4-methoxyphenyl)-4-methylthiazol-2-yl hexanamide;
(34) N-(5-(3-(N-(2-morpholinoethyl)aminosulfonyl))-4-methoxyphenyl)-4-methylthiazol-2-yl hexanamide;
(35) N-(5-(3-(((4-fluorophenyl)-12-nitridoacyl)sulfonyl)-4-methoxyphenyl)-4-methylthiazol-2-yl)hexanamide;
(36) N-(5-(3-(((4-chlorophenyl)-12-nitren)sulfonyl)-4-methoxyphenyl)-4-methylthiazol-2-yl)hexanamide;
(37) N-(5-(3-(((4-fluorobenzyl)-12-imino)sulfonyl)-4-methoxyphenyl)-4-methylthiazol-2-yl)hexanamide;
(38) N-(5-(3-(((3-fluorobenzyl)-12-nitren)sulfonyl)-4-methoxyphenyl)-4-methylthiazol-2-yl)hexanamide;
(39) N-(5-(3-(((2-fluorobenzyl)-12-nitren)sulfonyl)-4-methoxyphenyl)-4-methylthiazol-2-yl)hexanamide;
(40) N-(4-(3-(((2,4-difluorobenzyl)-12-nitren)sulfonyl)-4-methoxyphenyl)-5-methylthiazol-2-yl)hexanamide;
(41) N-(5-(3-(((3,4-difluorobenzyl)-12-nitren)sulfonyl)-4-methoxyphenyl)-4-methylthiazol-2-yl)hexanamide;
(42) N-(5-(3-(((3-chloro-4-fluorobenzyl)-12-nitren)sulfonyl)-4-methoxyphenyl)-4-methylthiazol-2-yl)hexanamide;
(43) N-(5-(3-(((4-fluoro-3-(trifluoromethyl)benzyl)-)benzyl)-12-nitren)sulfonyl)-4-methoxyphenyl)-4-methylthiazol-2-yl)hexanamide;
(44) N-(5-(4-methoxy-3-(((3.4.5-trifluorobenzyl)-12-nitren)sulfonyl)phenyl)-4-methylthiazol-2-yl)hexanamide;
(45) N-(5-(3-(((4-fluorophenethyl)-12-nitren)sulfonyl)-4-methoxyphenyl)-4-methylthiazol-2-yl)hexanamide;
(46) tert-butyl (2-(2-hexanamido-4-methylthiazol-5-yl)-2-methoxybenzamido)ethyl carbamate;
(47) N-(4-(3-(((2,4-difluorobenzyl)-12-nitren)sulfonyl)-4-methoxyphenyl)-5-methylthiazol-2-yl)hexanamide.

5. A method for preparing the inhibitor according to claim 1, comprising: reacting a starting material 1a, 1-(4-chlorophenyl)-2-acetone, or a starting material 1b, 1-(4-methoxyphenyl)-2-acetone, with chlorosulfonic acid to effect a meta-substitution reaction on the benzene ring, thereby obtaining an intermediate 2a or 2b, respectively; subjecting the intermediate 2a or 2b to a Hinsberg reaction to introduce an R₂ group, thereby obtaining intermediates 3a to 3g, respectively; subjecting the intermediates 3a to 3g and phenyltrimethylammonium tribromide to α-bromination, to obtain intermediates 4a to 4g, respectively; and condensing the intermediates 4a to 4g with N-acetylthiourea, to afford target compounds 5a to 5g, respectively, with a synthetic route depicted below:

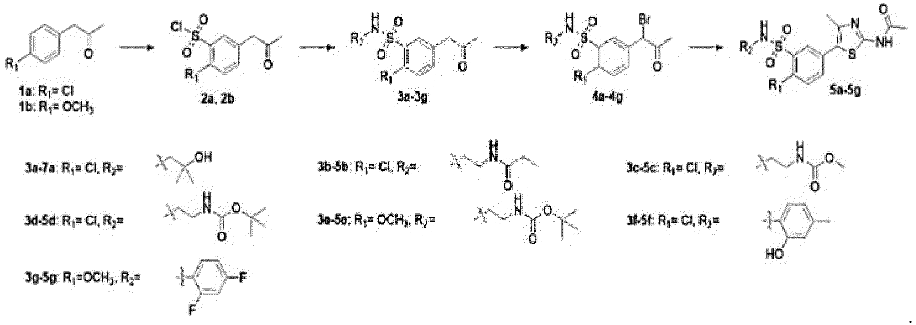

6. The method according to claim 5, further comprising: based on the method for preparing the target compounds 5a to 5g, reacting the intermediate 4d or 4e as a starting material with N-substituted thiourea as a further starting material, which is obtained by subjecting thiourea and a variety of acyl chlorides to a substitution reaction to introduce an R₃ group, to afford target compounds 7a to 7r, respectively, or condensing the intermediate 4e as a starting material with thiourea to obtain an intermediate 8 and subjecting the intermediate 8 and 3-acetamidopropanoyl chloride or 6-oxoheptanoyl chloride or 5-oxohexanoyl chloride to a substitution reaction, to afford target compounds 9a to 9c, respectively, with synthetic routes depicted below: 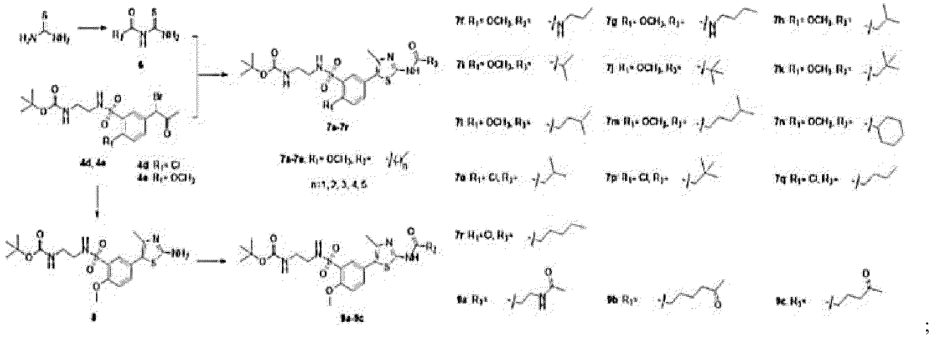 or based on the method for preparing the target compounds 5a to 5g, preparing intermediates 4h to 4x as starting materials and then condensing the intermediates 4h to 4x with N-hexanoylthiourea, to afford target compounds 10a to 10q, respectively, with a synthetic route depicted below:

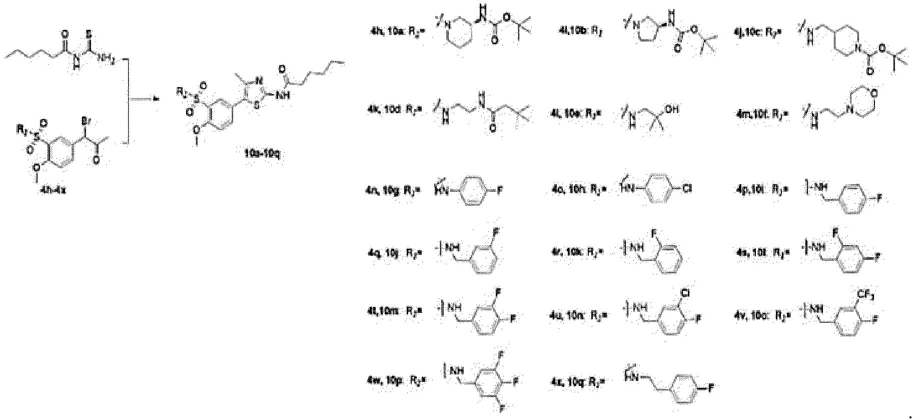

7. The method according to claim 6, further comprising: subjecting the intermediate 8 as a starting material to two sequential reactions to obtain an intermediate 9, subjecting the intermediate 9 and iron powder to a reduction reaction in refluxing acetic acid under heating conditions to obtain an intermediate 10, hydrolyzing the intermediate 10 under acidic conditions to obtain an intermediate 11, reacting the intermediate 11 with tert-butyl (2-aminoethyl)carbamate to obtain an intermediate 12, and subjecting the intermediate 12 to two sequential reactions to afford a target compound 46, with a synthetic route depicted below: 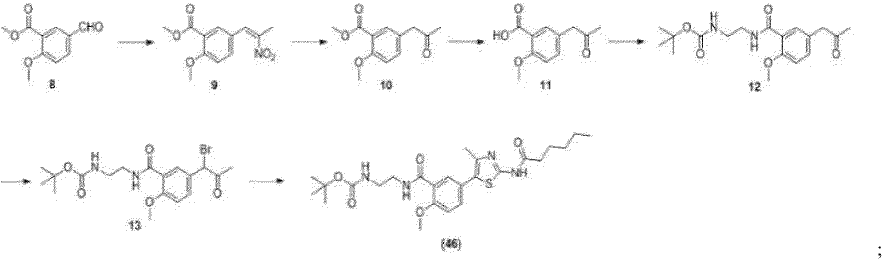 or subjecting 4'-methoxypropiophenone 14 as a starting material to four sequential reactions to afford a target compound 47, with a synthetic route depicted below:

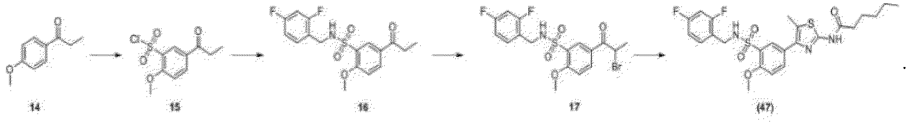

8. A pharmaceutical composition, comprising at least one pharmaceutically acceptable excipient, adjuvant or carrier, and the phenylthiazolamine-based PI4KIIIβ inhibitor according to any one of claims 1 to 3.

9. Use of the phenylthiazolamine-based PI4KIIIβ inhibitor according to claim 1 or the pharmaceutical composition according to claim 8 in the preparation of a medicament for preventing, treating or adjunctively treating a proliferative disease, metabolic disease, neurological disorder or tuberous sclerosis complex caused by PI4KIIIβ hyperactivation.

10. Use of the phenylthiazolamine-based PI4KIIIβ inhibitor according to claim 1 or the pharmaceutical composition according to claim 8 in the preparation of a medicament for inhibiting growth of cancer cells.
